# EUROPEAN PATENT APPLICATION

(11) **EP 3 725 872 A1**
(43) Date of publication of application: **21.10.2020**
(21) Application number: 18887677.5
(22) Date of filing: 10.10.2018
(51) Int. Cl.: C12M 3/00

(54) **CELL ENCAPSULATION DEVICE WITH SUCTION TUBE AND USE THEREOF**

(30) Priority: 12.12.2017 JP 2017238105
(71) Applicant: NATIONAL AGRICULTURE AND FOOD RESEARCH ORGANIZATION, Tsukuba-shi, Ibaraki 305-8517 (JP)
(72) Inventor: TAKEZAWA Toshiaki, Tsukuba-shi, Ibaraki 305-8634 (JP)
(74) Representative: V.O.
(86) International application number: PCT/JP2018/037711
(87) International publication number: WO 2019/116699

(57) **Abstract**

A cell encapsulation device with a suction tube includes a cell encapsulation device for constructing a multicellular structure formed by culturing cells, the cell encapsulation device being provided with a porous membrane on at least one of a top surface and a bottom surface and a frame member at a side surface, and a suction tube having a first tubular member and a second tubular member, in which, a first through hole and a second through hole are formed in an outer circumferential surface of the frame member, the first through hole and the second through hole communicating with one end portion of the first tubular member and one end portion of the second tubular member, respectively, the other end portion of the first tubular member has a connection mechanism to a suction means at the other end portion thereof, and the other end portion of the second tubular member is open to the outside.

## Description

### [Technical Field]

The present invention relates to a cell encapsulation device with a suction tube and uses thereof. Specifically, the present invention relates to a cell encapsulation device with a suction tube, a set for a cell encapsulation device with a suction tube, and a method for encapsulating cells. Priority is claimed on Japanese Patent Application No. 2017-238105 filed on December 12, 2017, the content of which is incorporated herein by reference.

### [Background Art]

Enterprises involved in the discovery of drugs and alternative methods to animal experiments purchase frozen cells from a cell bank or the like, then cryopreserve the sub-cultured and proliferated cells, and also prepare culture models using some of the cells in order to carry out the tests necessary to development. In other words, large amounts of money and time are consumed before the tests necessary for development are carried out. Furthermore, "tissue-type culture models" which are closer to living bodies, rather than monolayer culture cells, are required.

Examples of techniques related to "tissue-type culture models" include, for example, various gel embedding culturing techniques, spheroid culturing techniques (refer to, for example, Patent Document 1), various chamber culturing techniques (refer to, for example, Patent Document 2), and the like.

### [Citation List]

### [Patent Literature]

[Patent Document 1] Japanese Unexamined Patent Application, First Publication No. 2012-65555
[Patent Document 2] PCT International Publication No. WO2008/130025
[Patent Document 3] Japanese Unexamined Patent Application, First Publication No. 2001-149763
[Patent Document 4] PCT International Publication No. WO2012/026531
[Patent Document 5] Japanese Unexamined Patent Application, First Publication No. 2012-115262
[Patent Document 6] Japanese Unexamined Patent Application, First Publication No. 2015-035978

### [Summary of Invention]

### [Technical Problem]

The techniques of the related art related to the "tissue-type culture model" described in Patent Documents 1 and 2 are all complicated culturing operations and require time and money to construct the tissue-type culture models. Not only this, but there was also a difference between lots because the production reproducibility was not always good. Furthermore, culturing techniques in which cells are in an exposed state, such as spheroid culturing techniques, do not always have a good protection performance for the individual cells forming three-dimensional tissue.

In addition, previously, the present inventors developed a cell encapsulation device for constructing a tissue-type culture model. Cells were encapsulated in the device using a catheter with an indwelling needle or the like, but since this operation is complicated and requires skill, there was a demand for further improvement.

The present invention was made in view of the circumstances described above and provides a cell encapsulation device with a suction tube which is able to easily encapsulate cells by suctioning a suspension of cells, which has excellent cell protection performance, which is easy to handle, and which is able to culture cells for long periods.

### [Solution to Problem]

That is, the present invention includes the following aspects.

A cell encapsulation device with a suction tube according to a first aspect of the present invention includes a cell encapsulation device for constructing a multicellular structure formed by culturing cells, the cell encapsulation device being provided with a porous membrane on at least one of a top surface and a bottom surface and a frame member at a side surface, and a suction tube having a first tubular member and a second tubular member, in which, a first through hole and a second through hole are formed in an outer circumferential surface of the frame member, the first through hole and the second through hole communicating with one end portion of the first tubular member and one end portion of the second tubular member, respectively, the other end portion of the first tubular member has a connection mechanism to a suction means at the other end portion thereof, and the other end portion of the second tubular member is open to the outside.

In the cell encapsulation device with a suction tube according to the first aspect described above, the first tubular member and the second tubular member may be arranged to face each other, via the cell encapsulation device.

In the cell encapsulation device with a suction tube according to the first aspect described above, the cell encapsulation device may inject multiple cells suspended in a culture medium and an internal volume may be 10 mL or less.

In the cell encapsulation device with a suction tube according to the first aspect described above, the porous membrane may be a semipermeable membrane having liquid tightness in a gas phase and a semipermeable property in a liquid phase.

In the cell encapsulation device with a suction tube according to the first aspect described above, the semipermeable membrane may include a material having biocompatibility.

In the cell encapsulation device with a suction tube according to the first aspect described above, the material having biocompatibility may be a gelling component derived from extracellular matrices.

In the cell encapsulation device with a suction tube according to the first aspect described above, the gelling component derived from extracellular matrices may be native collagen or atelocollagen.

In the cell encapsulation device with a suction tube according to the first aspect described above, a film having air tightness may be further attachable to and detachable from an outer surface of the porous membrane.

In the cell encapsulation device with a suction tube according to the first aspect described above, the connection mechanism may have a filter.

A set for a cell encapsulation device with a suction tube according to a second aspect of the present invention includes the cell encapsulation device with a suction tube according to the first aspect of the present invention, and a rack accommodating the cell encapsulation device with a suction tube.

In the set for a cell encapsulation device with a suction tube according to the second aspect described above, the rack may have a U-shaped locking portion.

A method for encapsulating cells according to a third aspect of the present invention uses the cell encapsulation device with a suction tube according to the first aspect described above.

### [Advantageous Effects of Invention]

According to the cell encapsulation device with a suction tube of the aspect described above, it is possible to easily encapsulate cells by suctioning a suspension of cells, the cell protection performance is excellent, handling is easy, and it is possible to culture cells for long periods. According to the set for a cell encapsulation device with a suction tube of the aspect described above, it is possible to efficiently perform continuous production of cell encapsulation devices. According to the method for encapsulating cells of the aspect described above, it is possible to easily encapsulate cells in the device.

### [Brief Description of Drawings]

Fig. 1 is a front view showing an example of a cell encapsulation device with a suction tube of the present embodiment.
Fig. 2A is a cross-sectional view showing a cell encapsulation device with a suction tube according to a first embodiment of the present invention.
Fig. 2B is a cross-sectional view showing a cell encapsulation device with a suction tube according to a second embodiment of the present invention.
Fig. 2C is a cross-sectional view showing a cell encapsulation device with a suction tube according to a third embodiment of the present invention.
Fig. 3 is a perspective view and a side view showing an example of a set for a cell encapsulation device with a suction tube of the present embodiment.
Fig. 4A is an image showing a state in which a culture medium is suctioned by a cell encapsulation device with a suction tube (Vitrigel (registered trademark)-silicon ring-Vitrigel (registered trademark)) in Production Example 1.
Fig. 4B is an image showing a state in which a gel-loading tip is removed from the cell encapsulation device with a suction tube (Vitrigel (registered trademark)-silicon ring-Vitrigel (registered trademark)) in Production Example 1.
Fig. 4C is an image showing a state in which the cell encapsulation device with a suction tube (Vitrigel (registered trademark)-silicon ring-Vitrigel (registered trademark)) in Production Example 1 is submerged in a 24-well plate.
Fig. 5A is an image showing a state in which a culture medium is suctioned by a cell encapsulation device with a suction tube (Vitrigel (registered trademark)-silicon ring-filter) in Production Example 2.
Fig. 5B is an image showing a state in which a PET film and a gel-loading tip are removed from a cell encapsulation device with a suction tube (Vitrigel (registered trademark)-silicon ring-filter) in Production Example 2.
Fig. 5C is an image showing a state in which the cell encapsulation device with a suction tube (Vitrigel (registered trademark)-silicon ring-filter) in Production Example 2 is submerged in a 24-well plate.
Fig. 6A is an image showing a state in which a culture medium is suctioned by a cell encapsulation device with a suction tube (filter-silicon ring-filter) in Production Example 3.
Fig. 6B is an image showing a state in which the PET film and the gel-loading tip are removed from the cell encapsulation device with a suction tube (filter-silicon ring-filter) in Production Example 3.
Fig. 6C is an image showing a state in which the cell encapsulation device with a suction tube(filter-silicon ring-filter) in Production Example 3 is submerged in a 24-well plate.
Fig. 7 is an image showing a spheroid produced using a cell encapsulation device (plastic ring-filter) obtained in Production Example 4 in Example 1. The scale bar indicates 100 µm.

### [Description of Embodiments]

### <Cell Encapsulation Device with Suction Tube>

A detailed description will be given below of the cell encapsulation device with a suction tube of the present embodiment with reference to the drawings.

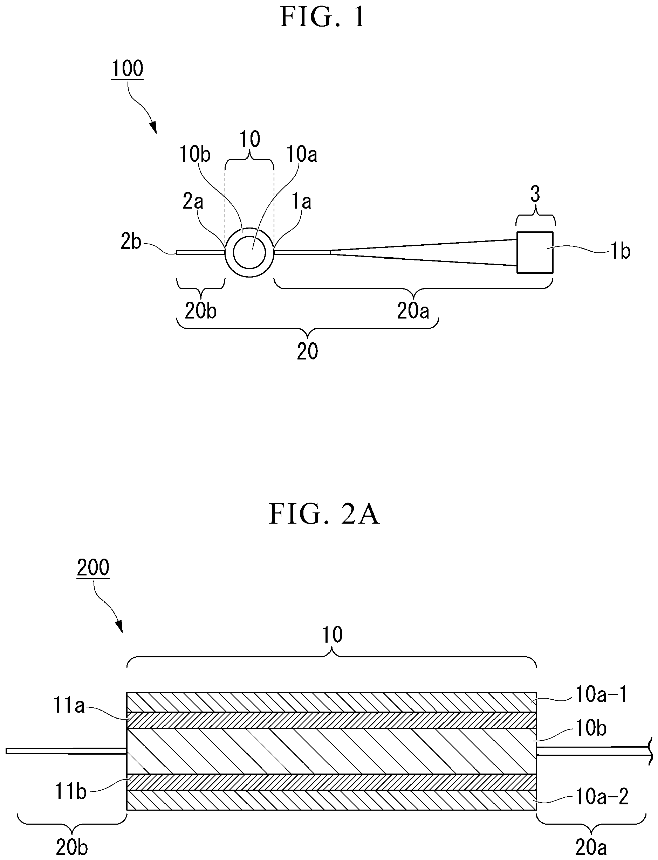

Fig. 1 is a front view showing an example of the cell encapsulation device with a suction tube of the present embodiment.

A cell encapsulation device with a suction tube 100 shown in Fig. 1 is provided with a cell encapsulation device 10 and a suction tube 20.

The cell encapsulation device 10 is provided with a porous membrane 10a on at least one of a top surface and a bottom surface and is provided with a frame member 10b on a side surface. In addition, the cell encapsulation device 10 is for constructing a multicellular structure formed by culturing cells.

The suction tube 20 has a first tubular member 20a and a second tubular member 20b.

A first through hole and a second through hole in communication with one end portion 1a of the first tubular member 20a and one end portion 2a of the second tubular member 20b, respectively, are formed on the outer circumferential surface of the frame member 10b of the cell encapsulation device 10.

The first tubular member 20a has a connection mechanism 3 for a suction means at another end portion 1b.

Another end portion 2b of the second tubular member 20b is open to the outside.

The positions of the first tubular member 20a and the second tubular member 20b in the frame member 10b are not particularly limited; however, as shown in Fig. 1, the first tubular member 20a and the second tubular member 20b are preferably arranged so as to face each other, via the cell encapsulation device 10. Due to this, when the cell encapsulation device with a suction tube and a suction mechanism are connected, it is possible to easily suction a suspension of cells by the suction force of the suction mechanism and encapsulate the suspension of cells in the cell encapsulation device.

In addition, the first tubular member 20a and the second tubular member 20b are inserted into the frame member 10b so as to be easily pulled out from the first through hole and the second through hole in the outer circumferential surface of the frame member 10b after cells are encapsulated in the cell encapsulation device 10 to close the through holes.

Providing the configuration described above makes it possible for the cell encapsulation device with a suction tube of the present embodiment to easily encapsulate cells. In addition, the cell encapsulation device with a suction tube of the present embodiment has excellent cell protection performance, is easy to handle, and is able to culture cells for long periods.

### <First Embodiment>

Fig. 2A is a cross-sectional view showing a cell encapsulation device with a suction tube according to the first embodiment of the present invention. A detailed description will be given below mainly of the structure of the cell encapsulation device 10 with reference to Fig. 2A. In the drawings after Fig. 2A, the same components as those shown in the previously explained diagrams are denoted by the same reference numerals as in the previously explained diagrams and detailed description thereof will be omitted. In addition, in Fig. 2A, the structure of the other end portion 1b of the first tubular member 20a is omitted to illustrate the structure of the cell encapsulation device 10 in detail.

In the cell encapsulation device 10 included in the cell encapsulation device with a suction tube 200 shown in Fig. 2A, a first adhesive layer 11a is laminated on a top surface of the frame member 10b, and a porous membrane 10a-1 of the top surface side is further laminated on the first adhesive layer 11a. Also, for the bottom surface of the frame member 10b, in the same manner, a second adhesive layer 11b is laminated below the bottom surface of the frame member 10b and a porous membrane 10a-2 of the bottom surface side is further laminated below the second adhesive layer 11b. That is, in the cell encapsulation device 10 shown in Fig. 2A, the porous membrane 10a-2 of the bottom surface side, the second adhesive layer 11b, the frame member 10b, the first adhesive layer 11a, and the porous membrane 10a-1 of the top surface side are laminated in this order.

In the cell encapsulation device with a suction tube 200, the porous membrane 10a-1 of the top surface side and the porous membrane 10a-2 of the bottom surface side may be formed of the same material as each other or formed of different materials. The material of the porous membrane is as described in "Porous Membrane" below.

It is possible to appropriately select the materials of the first adhesive layer 11a and the second adhesive layer 11b according to each material of the porous membrane 10a-1 of the top surface side, the porous membrane 10a-2 of the bottom surface side, and the frame member 10b which are to be adhered. In addition, the materials of the first adhesive layer 11a and the second adhesive layer 11b may be formed of the same material as each other or formed of different materials. The materials of the first adhesive layer 11a and the second adhesive layer 11b are as described in "Adhesive Layer" below.

It is possible to form the first adhesive layer 11a and the second adhesive layer 11b according to the shape of the frame member 10b. For example, in a case where the frame member 10b has a ring shape, the first adhesive layer 11a and the second adhesive layer 11b are formed to have a concentric circle shape on the frame member 10b and in a ring shape with a size in contact with the porous membrane 10a-1 of the top surface side or the porous membrane 10a-2 of the bottom surface side, and the frame member 10b.

### <Second Embodiment>

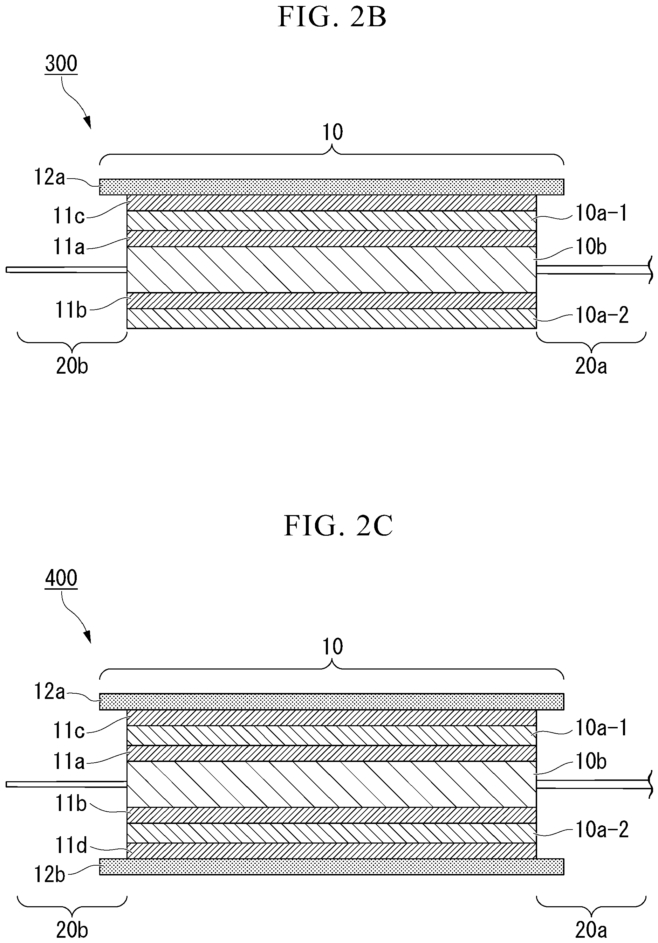

Fig. 2B is a cross-sectional view showing a cell encapsulation device with a suction tube according to the second embodiment of the present invention.

The cell encapsulation device 10 included in a cell encapsulation device with a suction tube 300 shown in Fig. 2B has the same configuration as the cell encapsulation device with a suction tube 200 shown in Fig. 2A except for the point that a third adhesive layer 11c and a film 12a having air tightness are provided, in addition to the cell encapsulation device with a suction tube 200 described above. That is, in the cell encapsulation device 10 shown in Fig. 2B, the porous membrane 10a-2 of the bottom surface side, the second adhesive layer 11b, the frame member 10b, the first adhesive layer 11a, the porous membrane 10a-1 of the top surface side, the third adhesive layer 11c, and the film 12a having air tightness are laminated in this order. In addition, the film 12a having air tightness is detachably attached to the outer surface of the porous membrane 10a-1 of the top surface side, via the third adhesive layer 11c.

In the cell encapsulation device with a suction tube 300, the materials of the porous membrane 10a-1 of the top surface side and the porous membrane 10a-2 of the bottom surface side are the same as in the cell encapsulation device with a suction tube 200 shown in Fig. 2A.

It is possible to appropriately select the materials of the first adhesive layer 11a, the second adhesive layer 11b, and the third adhesive layer 11c according to each material of the porous membrane 10a-1 of the top surface side, the porous membrane 10a-2 of the bottom surface side, the frame member 10b, and the film 12a having air tightness, which are to be adhered. In addition, the materials of the first adhesive layer 11a, the second adhesive layer 11b, and the third adhesive layer 11c may be formed of the same material as each other or formed of different materials. The materials of the first adhesive layer 11a, the second adhesive layer 11b, and the third adhesive layer 11c are as described in "Adhesive Layer" below.

The shapes of the first adhesive layer 11a and the second adhesive layer 11b are the same as in the cell encapsulation device with a suction tube 200 shown in Fig. 2A.

It is possible to form the third adhesive layer 11c according to the shape of the film 12a having air tightness. For example, in a case where the film 12a having air tightness has a circular shape, the third adhesive layer 11c is formed in a circular shape or ring shape with a size in contact with the porous membrane 10a-1 of the top surface side and the film 12a having air tightness, to have a concentric circle shape on the film 12a having air tightness.

### <Third Embodiment>

Fig. 2C is a cross-sectional view showing a cell encapsulation device with a suction tube according to a third embodiment of the present invention.

The cell encapsulation device 10 included in a cell encapsulation device with a suction tube 400 shown in Fig. 2C has the same configuration as the cell encapsulation device with a suction tube 300 shown in Fig. 2B except for the point that a fourth adhesive layer 11d and a film 12b having air tightness are provided, in addition to the cell encapsulation device with a suction tube 300 described above. That is, in the cell encapsulation device 10 shown in Fig. 2C, the film 12b having air tightness, the fourth adhesive layer 11d, the porous membrane 10a-2 of the bottom surface side, the second adhesive layer 11b, the frame member 10b, the first adhesive layer 11a, the porous membrane 10a-1 of the top surface side, the third adhesive layer 11c, and the film 12a having air tightness are laminated in this order. In addition, the film 12a having air tightness and the film 12b having air tightness are detachably attached to the outer surface of the porous membrane 10a-1 of the top surface side and the outer surface of the porous membrane 10a-2 of the bottom surface side, via the third adhesive layer 11c and the fourth adhesive layer 11d, respectively.

In the cell encapsulation device with a suction tube 400, the porous membrane 10a-1 of the top surface side and the porous membrane 10a-2 of the bottom surface side are the same as in the cell encapsulation device with a suction tube 200 shown in Fig. 2A.

It is possible to appropriately select the materials of the first adhesive layer 11a, the second adhesive layer 11b, the third adhesive layer 11c, and the fourth adhesive layer 11d according to each material of the porous membrane 10a-1 of the top surface side, the porous membrane 10a-2 of the bottom surface side, the frame member 10b, the film 12a having air tightness, and the film 12b having air tightness, which are to be adhered. In addition, the materials of the first adhesive layer 11a, the second adhesive layer 11b, the third adhesive layer 11c, and the fourth adhesive layer 11d may be formed of the same material as each other or formed of different materials. The materials of the first adhesive layer 11a, the second adhesive layer 11b, the third adhesive layer 11c, and the fourth adhesive layer 11d are as described in the "Adhesive Layer" below.

The shapes of the first adhesive layer 11a and the second adhesive layer 11b are the same as in the cell encapsulation device with a suction tube 200 shown in Fig. 2A.

The shape of the third adhesive layer 11c is the same as the cell encapsulation device with a suction tube 300 shown in Fig. 2B.

It is possible to form the fourth adhesive layer 11d according to the shape of the film 12b having air tightness. For example, in a case where the film 12b having air tightness has a circular shape, the fourth adhesive layer 11d is formed in a circular shape or ring shape with a size in contact with the porous membrane 10a-2 of the bottom surface side and the film 12b having air tightness, to have a concentric circle shape on the film 12b having air tightness.

The cell encapsulation device with a suction tube of the present embodiment is not limited to the devices shown in Fig. 1 and FIGs. 2A to 2C and may be a device in which parts of the configurations of devices shown in Fig. 1 and FIGs. 2A to 2C are modified or removed or a device in which another configuration is further added to the device described above, in a range in which the effect of the cell encapsulation device with a suction tube of the present embodiment is not impaired.

For example, in the cell encapsulation devices with a suction tube shown in Fig. 1 and FIGs. 2A to 2C, a device provided with a porous membrane on the top surface and the bottom surface is shown; however, the porous membrane may be provided on at least one of the top surface and the bottom surface. That is, the porous membrane may be provided only on the top surface, or may be provided only on the bottom surface. In a case where the cell encapsulation device with a suction tube is provided with a porous membrane only on either the top surface or the bottom surface, the other portions may be formed of the same material as the frame member, or may be formed of a different material.

For example, in the cell encapsulation devices with a suction tube shown in Fig. 1 and FIGs. 2A to 2C, the shape of the cell encapsulation device 10 is shown as a cylinder; however, it is also possible to use other shapes. The shape of the cell encapsulation device 10 may be any shape as long as it is possible to encapsulate cells and uniformly distribute oxygen and nutrients dissolved in a culture medium to the cells. In addition, in the cell encapsulation device 10, the inside of the device may be filled with a culture medium or a gas portion may be left without being filled with the culture medium. Specific examples of the shape of the cell encapsulation device 10 other than a ring-shaped cylinder include a cylinder with a hollow fiber-shape or the like, a circular cone, a circular truncated cone, a pyramid, a truncated pyramid, a sphere, a polyhedron (for example, a tetrahedron, a pentahedron, a hexahedron (including a cube), an octahedron, a dodecahedron, an icosahedron, an icositetrahedron, a Kepler-Poinsot polyhedron, or the like), and the like, without being limited thereto.

As shown in Fig. 1 and FIGs. 2A to 2C, in a case where the shape of the cell encapsulation device 10 in the cell encapsulation device with a suction tube is a ring-shaped cylinder, the inner diameter of the cell encapsulation device is preferably 1 mm or more and 60 mm or less, more preferably 3 mm or more and 35 mm or less, and even more preferably 5 mm or more and 26 mm or less.

In addition, the outer diameter of the cell encapsulation device 10 is preferably 3 mm or more and 68 mm or less, more preferably 5 mm or more and 43 mm or less, and even more preferably 7 mm or more and 32 mm or less.

In addition, the thickness (cylinder height) of the cell encapsulation device 10 is 5 µm or more, preferably 50 µm or more and 15 mm or less, more preferably 100 µm or more and 10 mm or less, and even more preferably 200 µm or more and 2.5 mm or less.

In the present specification, the "thickness (cylinder height) of the cell encapsulation device" means a distance from the edge portion on the outer side of the top surface of the cell encapsulation device to the edge portion on the outer side of the bottom surface.

For example, in the cell encapsulation device with a suction tube shown in Fig. 1 and FIGs. 2A to 2C, a cell encapsulation device in which the top surface and bottom surface are flat is shown; however, the top surface and bottom surface may be a concave structure or convex structure. In particular, when the top surface and the bottom surface have a concave structure, a certain distance (for example, 5 µm or more) is preferably maintained such that the central portion of the concave portion inside the top surface (the most concave portion inside the top surface) and the central portion of the concave portion inside the bottom surface (the most concave portion inside the bottom surface) are not in contact. Due to this, the thickness (cylinder height) of the cell encapsulation device, that is, the distance from the edge portion on the outside of the top surface of the cell encapsulation device to the edge portion on the outside of the bottom surface is longer than the distance from the central portion of the concave portion on the outside of the top surface (the most concave portion on the outside in the top surface) to the central portion of the concave portion on the outside of the bottom surface (the most concave portion on the outside in the bottom surface). Due to this, for example, in a case where medicine is added from the top surface, it is possible to maintain the directionality of the added medicine. Furthermore, since the top surface and the bottom surface have a concave structure, it is possible to newly seed cells on the outside of the top surface and the bottom surface to carry out culturing.

For example, in the cell encapsulation device with a suction tube shown in Fig. 1 and FIGs. 2A to 2C, a device in which the shapes of the first through hole and the second through hole in the outer circumferential surface of the frame member are circular was shown; however, the shapes may be other than circular as long as the first tubular member and the second tubular member are easily pulled out from the through hole to close the through holes.

Examples of the shapes of the first through hole and the second through hole other than circles include polygons (including regular polygons and the like), ellipses, and the like.

The diameters of the first through hole and the second through hole may be appropriately adjusted according to the thickness (that is, the height of the frame member) of the cell culturing device, and it is possible to set the diameters to, for example, 10 µm or more and 1000 µm or less.

For example, in the cell encapsulation device with a suction tube shown in Fig. 1 and FIGs. 2A to 2C, a method for adhering the porous membrane and the frame member via an adhesive layer was shown; however, the joining may be carried out by other joining methods.

Specific examples of a method for joining the porous membrane and the frame member other than the method for adhering via an adhesive layer include a method for joining by heat welding using a heat sealer or a hot plate, ultrasonic waves, a laser, or the like, a method using a mortise joint for joining by producing a tenon and a mortise, and the like, without being limited thereto. Examples of methods using a mortise joint include single-sided, double-sided, three-sided, four-sided, small rooted, shoulder-tenon, two-tenon, two-step tenon, or the like.

In addition, one type of these joining methods may be used or two types or more may be used in combination.

For example, in the cell encapsulation device with a suction tube shown in Fig. 1 and FIGs. 2A to 2C, it is possible for the frame member 10b to have a support. The frame member 10b having a support makes it possible to culture the cells in a gas phase by, for example, fixing the cell encapsulation device 10 in which the cells are encapsulated in a slightly larger container. In addition, the frame member 10b having a support, for example, makes it possible for the cell encapsulation device 10 in which cells are encapsulated to float in a culture medium due to buoyancy. In addition, in a case where the porous membrane 10a-1 of the top surface side and the porous membrane 10a-2 of the bottom surface side are provided as in the cell encapsulation device 10 shown in FIGs. 2A to 2C, since it is also possible to place the top surface to be in contact with air and the bottom surface to be in contact with the culture medium, it is possible to culture the cells in the gas phase and the liquid phase.

In addition, the support may be fixed to the frame member 10b or may be removable.

In addition, in the cell encapsulation device with a suction tube of the present embodiment, it is possible to freely adjust the size and shape of each component (porous membrane, frame member, suction tube, and the like) according to the purpose.

A detailed description will be given below of each component included in the cell encapsulation device with a suction tube of the present embodiment.

### <Cell Encapsulation Device>

The cell encapsulation device is provided with a porous membrane on at least one of the top surface and the bottom surface, and a frame member on the side surface.

It is sufficient if the internal volume of the cell encapsulation device is small scale and it is possible to inject multiple cells suspended in a culture medium and to construct a multicellular structure used in an in vitro test system such as a test for assaying cell activity. Specifically, for example, the internal volume is preferably 10 mL or less, more preferably 10 µL or more and 5 mL or less, even more preferably 15 µL or more and 2 mL or less, and particularly preferably 20 µL or more and 1 mL or less. Setting the internal volume to the upper limit or less makes it possible to sufficiently supply oxygen and culture medium nutrients and to efficiently culture the cells for a long period of time. In addition, setting the internal volume to the lower limit or more makes it possible to obtain cells having a sufficient cell number and cell density for use in an in vitro test system.

### [Porous Membrane]

In the present specification, the term "porous membrane" means a membrane having a large number of pores, and encompasses membranes having voids and membranes having pores and voids.

The porous membrane used in the cell encapsulation device of the present embodiment is not particularly limited as long as it is a membrane having holes to the extent that the cells encapsulated in the inside do not permeate to the outside. Examples of the porous membrane include filter paper, semipermeable membranes (for example, ultrafiltration membranes, and the like), nonwoven fabric, gauze-like mesh, various membrane filters, and the like, without being limited thereto.

In addition, it is possible to set the size of the pores of the porous membrane, for example, 0.01 µm or more and 1,500 µm or less, 0.01 µm or more and 1.0 µm or less, or 0.01 µm or more and 0.45 µm or less. The size of the holes may be appropriately selected according to the size of the cells to be encapsulated therein or the size of a small living individual described below.

The thickness of the porous membrane is not particularly limited, but is preferably 1 µm or more and 1000 µm or less, more preferably 1 µm or more and 500 µm or less, even more preferably 5 µm or more and 300 µm or less, and particularly preferably 10 µm or more and 200 µm or less. Setting the thickness of the porous membrane within the above range makes it possible to obtain strength at which it is possible to inject the cells into the cell encapsulation device and carry out culturing. In addition, in a case where the porous membrane is a semipermeable membrane, setting the thickness within the above range makes it possible to suitably use the cell encapsulation device of the present embodiment as a medical cell transplantation device.

Here, the "thickness of the porous membrane" means the thickness of the entire porous membrane, for example, the thickness of a porous membrane formed of a plurality of layers means the thickness of the sum of all the layers forming the porous membrane.

In particular, the porous membrane in the present embodiment is preferably various membrane filters. Since the pore size of the membrane filters is relatively large, the membrane filters do not have liquid tightness; however, there is a large amount of oxygen permeation and it is possible for the cells encapsulated inside to proliferate with high efficiency.

In a case where the porous membrane on either the top surface or the bottom surface has pores of approximately 0.1 µm or more, such as a membrane filter, it is preferable to have a configuration having a film having air tightness described below, as in the cell encapsulation devices with a suction tube 300 and 400 shown in Fig. 2B and Fig. 2C. This is to prevent difficulty in suctioning the suspension of cells as neither air tightness nor liquid tightness are maintained in the inside of the cell encapsulation device when the pores of the porous membrane are as large as approximately 0.1 µm or more. In a case where the porous membrane on either the top surface or the bottom surface has pores of approximately 0.1 µm or more, such as a membrane filter, having a film having air tightness secures the air tightness inside the cell encapsulation device to some extent and makes it possible to easily suction the suspension of cells.

For example, in a case where the porous membrane has pores of approximately 0.45 µm or less, such as a membrane filter, and the cell encapsulation device of the present embodiment in which cells are encapsulated is placed in a container including a culture medium, the cells in the cell encapsulation device do not permeate outside the device. On the other hand, it is possible to allow nutrients dissolved in the culture medium to permeate through the inside of the cell encapsulation device and to allow cell products including waste products dissolved in the culture medium to permeate outside the cell encapsulation device. For this reason, it is possible to use the cell encapsulation device of the present embodiment in the culturing of cells for long periods.

Alternatively, in particular, the porous membrane in the present embodiment is preferably a semipermeable membrane having liquid tightness in a gas phase and a semipermeable property in a liquid phase. Since the semipermeable membrane has liquid tightness in the gas phase, for example, in a case where a liquid such as a culture medium is included inside the cell encapsulation device, it is possible to maintain the liquid inside without leaking in the gas phase. This liquid tightness is due to the surface tension on the semipermeable membrane. On the other hand, since it is possible for gas to pass through, in a case where a liquid is included inside, the liquid inside evaporates over time.

In a case where the porous membrane is a semipermeable membrane and has liquid tightness, even if the configuration does not have a film having air tightness described below, as in the cell encapsulation device with a suction tube 200 shown in Fig. 2A, since the inside of the cell encapsulation device has a certain degree of air tightness, it is possible to easily suction the suspension of cells.

In addition, since the semipermeable membrane used in the cell encapsulation device has a semipermeable property in a liquid phase, for example, in a case where the cell encapsulation device of the present embodiment in which cells are encapsulated is placed in a container including a culture medium, the cells in the cell encapsulation device do not permeate to the outside of the device, while it is possible for the nutrients dissolved in the culture medium to permeate to the inside of the cell encapsulation device, and for cell products including waste products dissolved in the culture medium to permeate to the outside of the cell encapsulation device. For this reason, it is possible to use the cell encapsulation device of the present embodiment in the culturing of cells for long periods.

More specifically, the semipermeable membrane used in the cell encapsulation device of the present embodiment may be, for example, any semipermeable membrane through which it is possible for a high molecular compound having a molecular weight of approximately 1,000,000 or less to permeate, for example, a semipermeable membrane through which it is possible for a molecular compound having a molecular weight of approximately 200,000 or less to permeate.

In the present specification, "liquid tightness" means a state in which liquid does not leak.

In the present specification, "semipermeable" means a property with which it is possible for only molecules or ions having a certain molecular weight or less to permeate and a "semipermeable membrane" is a membrane having this property.

The material of the porous membrane may be any material having no cytotoxicity, may be a natural polymer compound, or may be a synthetic polymer compound. In addition, in a case where the porous membrane is a semipermeable membrane, the material preferably includes a material having biocompatibility, and more preferably includes a material having bio-absorbability. In a case where the entire cell encapsulation device of the present embodiment is formed of a semipermeable membrane having biocompatibility or bio-absorbability, use is possible as a medical cell transplantation device.

In the present specification, "biocompatibility" means an evaluation criterion indicating compatibility between living tissue and the material. In addition, "having biocompatibility" means a state where the material itself has no toxicity, has no components derived from microorganisms such as endotoxins, does not physically stimulate living tissue, and is not rejected even when interacting with proteins, cells, or the like forming living tissue. In addition, in the present specification, "bio-absorbability" means a property by which a material retains a shape or physical properties for a certain period of time in a living body, and then decomposes and is absorbed to disappear from the introduction portion into the living body.

Examples of the natural polymer compound include a gelling component derived from extracellular matrices, polysaccharide, chitin, poly(3-hydroxyalkanoate) (particularly, poly(β-hydroxybutyrate) and poly(3-hydroxyoctanoate)), poly(3-hydroxy fatty acid), fibrin, agar, agarose, and the like, without being limited thereto. Examples of polysaccharides include alginate, cellulose, dextran, pullulane, polyhyaluronic acid, and derivatives or the like thereof.

The cellulose includes cellulose modified by synthesis and examples thereof include cellulose derivatives and the like. Specific examples of the cellulose derivative include alkyl cellulose, hydroxyalkyl cellulose, cellulose ether, cellulose ester, nitrocellulose, chitosan, and the like. More specific examples of cellulose derivatives include methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose, cellulose triacetate, cellulose sulfate sodium salt, and the like.

In particular, the natural polymer compound is preferably a gelling component derived from extracellular matrices, fibrin, agar, or agarose due to having excellent water retention.

Examples of gelling components derived from extracellular matrices include collagen (type I, II, III, V, XI, and the like), basement membrane components (trade name: Matrigel) reconstitured from a mouse EHS tumor extract (containing type IV collagen, laminin, heparan sulfate proteoglycan, or the like), glycosaminoglycan, hyaluronic acid, proteoglycan, gelatin, and the like, without being limited thereto. It is possible to produce a porous membrane (particularly, a semipermeable membrane) by selecting the components, such as a salt, the concentration, the pH, and the like, which are optimal for each gelation. In addition, combining the raw materials makes it possible to obtain a porous membrane (particularly, a semipermeable membrane) imitating various in vivo tissues.

Examples of the synthetic polymer compound include polyphosphazene, poly(vinyl alcohol), polyamide (for example, nylon, and the like), polyesteramide, poly(amino acid), polyanhydride, polysulfone, polycarbonate, polyacrylate (acrylic resin), polyalkylene (for example, polyethylene or the like), polyacrylamide, polyalkylene glycol (for example, polyethylene glycol or the like), polyalkylene oxide (for example, polyethylene oxide or the like), polyalkylene terephthalate (for example, polyethylene terephthalate or the like), polyorthoester, polyvinyl ether, polyvinyl ester, polyvinyl halide, polyvinyl pyrrolidone, polyester, polysiloxane, polyurethane, polyhydroxy acid (for example, polylactide, polyglycolide, and the like), poly(hydroxybutyric acid), poly(hydroxyvaleric acid), poly[lactide-co-(ε-caprolactone)], poly[glycolide-co-(ε-caprolactone)], and the like), poly(hydroxyalkanoate), and copolymers thereof, and the like, without being limited thereto.

More specific examples of the polyacrylate (acrylic resin), include poly(methyl methacrylate), poly(ethyl methacrylate), poly(butyl methacrylate), poly(isobutyl methacrylate), poly(hexyl methacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate), and the like.

In particular, as the synthetic polymer compound, due to having bio-absorbability, polyhydroxy acids (for example, polylactide, polyglycolide, and the like), polyethylene terephthalate, poly(hydroxybutyric acid), poly(hydroxyvaleric acid), poly[lactide-co-(ε-caprolactone)], poly[glycolide-co-(ε-caprolactone)], and the like), poly(hydroxyalkanoate), polyorthoester, or copolymers thereof are preferable.

The material of the porous membrane may be formed of one type of the materials shown above or may be formed of two types or more. In addition, the material of the porous membrane may be formed of either a natural polymer compound or a synthetic polymer compound, or may be formed of both a natural polymer compound and a synthetic polymer compound.

In addition, in a case where the porous membrane is formed of two types or more of the materials shown above, the porous membrane may be formed of a mixture of the materials shown above. Alternatively, the porous membrane may be formed by laminating two layers or more of porous membranes formed of one type of material, and the materials forming each porous membrane may be formed of films which are different from each other.

In particular, in a case where the porous membrane in the present embodiment is a membrane filter, the material is preferably a synthetic polymer compound, more preferably a polyamide, and even more preferably nylon.

In particular, in a case where the porous membrane in the present embodiment is a semipermeable membrane, the material is preferably a natural polymer compound, more preferably a gelling component derived from extracellular matrices, and even more preferably collagen. In addition, as a more preferable raw material among collagens, it is possible to illustrate native collagen or atelocollagen.

In a case where the material of the porous membrane in the present embodiment is an extracellular matrix-derived component, the extracellular matrix-derived component is preferably contained as 0.1 mg or more and 10.0 mg or less per unit area of 1 cm² of the porous membrane (particularly, a semipermeable membrane), and more preferably contained as 0.5 mg or more and 5.0 mg or less. In particular, in a case where the extracellular matrix-derived component is atelocollagen, the atelocollagen is preferably contained as 0.5 mg or more and 10.0 mg or less per unit area of 1 cm² of the porous membrane (particularly, a semipermeable membrane), and more preferably contained as 2.5 mg or more and 5.0 mg or less per 1 cm².

When the content of the extracellular matrix-derived component (particularly, atelocollagen) in the porous membrane (particularly, a semipermeable membrane) is in the above range, it is possible to obtain a strength at which it is possible to inject the cells into the cell encapsulation device and carry out culturing.

The "mass of the membrane per unit area of 1 cm²" refers to the mass of the component contained per 1 cm² of the material piece, with the thickness of the membrane not being limited.

In addition, there is no concern that the porous membrane in the present embodiment will break when used and the porous membrane is extremely excellent in practical use. In particular, in a case of being used for a medical cell transplantation device, the strength of the porous membrane (particularly, a semipermeable membrane) has strength enough to withstand a transplantation operation.

### (Method for Manufacturing Porous Membrane)

### 1. Method for Manufacturing Porous Membrane using Synthetic Polymer Compound

For example, in a case where the constituent material of the porous membrane is the synthetic polymer compound, it is possible to manufacture the porous membrane using a known method (for example, refer to Patent Document 3 and the like).

More specifically, as a method for manufacturing a porous membrane using the synthetic polymer compound, first, a membrane-producing solution in which the synthetic polymer compound is dissolved in an organic solvent is prepared.

The organic solvent may be any good solvent for the synthetic polymer compound and examples thereof include tetrahydrofuran, dioxane, dimethylformamide, dimethylacetamide, N-methyl-2-pyrrolidone, and the like, without being limited thereto.

The mixing ratio of the synthetic polymer compound and the organic solvent may be appropriately adjusted according to the types of the synthetic polymer compound and the organic solvent to be used, for example, the ratio may be 15% by mass of the synthetic polymer compound and 85% by mass of the organic solvent.

In addition, the temperature of the organic solvent at the time of dissolution may be generally 30°C or higher and 100°C or lower, preferably 50°C or higher and 80°C or lower.

Next, for example, using a method such as discharging the prepared membrane-producing solution from a nozzle and causing coagulation in a coagulating liquid, a porous membrane having a predetermined shape is produced.

As the coagulating liquid, it is preferable to use a mixed liquid of an organic solvent and water. As an organic solvent used for the coagulating liquid, it is possible to use the same organic solvents shown as the organic solvent used for dissolving the synthetic polymer compound. The organic solvent used for the coagulating liquid may be the same type as the organic solvent used for dissolving the synthetic polymer compound, or may be a different type.

In addition, the ratio of water in the coagulating liquid may be, for example, 30% by mass or more and 80% by mass or less.

Furthermore, for the purpose of adjusting the coagulation rate, for example, alcohols such as methanol, ethanol, isopropanol and glycerin and glycols such as ethylene glycol and propylene glycol may be added to the coagulating liquid.

The obtained porous membrane may be washed with distilled water or the like, and further sterilized by ultraviolet irradiation or the like for use.

### 2. Method for Manufacturing Porous Membrane using Hydrogel

In addition, for example, in a case where the constituent material of the porous membrane is a hydrogel, manufacturing is possible using a known method (for example, refer to Patent Documents 4 to 6 and the like).

In the present specification, the term "hydrogel" refers to a substance in which a polymer compound has a network structure due to chemical bonding and has a large amount of water in the network. More specifically, the hydrogel means a gel which is gelled by introducing cross-linking into an artificial material of a natural polymer compound or a synthetic polymer compound.

Examples of hydrogels include natural polymer compounds such as the gelling component derived from extracellular matrices described above, fibrin, agar, agarose, and cellulose, and synthetic polymer compounds such as polyacrylamide, polyvinyl alcohol, polyethylene oxide, and poly(II-hydroxyethylmethacrylate)/polycaprolactone.

More specifically, as a method for manufacturing a porous membrane using a hydrogel, first, a hydrogel in a state of not being completely gelled (may be referred to below as a "sol") is arranged in a mold and gelling is induced.

In a case where the sol is a collagen sol, a collagen sol having an optimal salt concentration may be prepared for use using physiological saline, phosphate buffered saline (PBS), Hank's Balanced Salt Solution (HBSS), a basic culture medium, a serum-free culture medium, a serum-containing culture medium, or the like. In addition, the pH of the solution at the time of collagen gelation may be 6 or more and 8 or less, for example.

In particular, in a case where a serum-free culture medium is used, since it is possible to avoid including substances (for example, antigens, pathogenic factors, and the like) included in other animal serum components which are not suitable for transplantation in the porous membrane, it is possible to obtain a porous membrane (in particular, a semipermeable membrane) suitable for a case of being used for a medical cell transplantation device.

In addition, the preparation of the collagen sol may be performed, for example, at approximately 4°C. After that, the maintained temperature during gelation may be set to a temperature lower than the denaturation temperature of the collagen depending on the type of animal of the collagen to be used, generally, it is possible to perform the gelling for several minutes to several hours by maintaining the temperature at 20°C or higher and 37°C or lower.

In addition, the concentration of the collagen sol for producing the porous membrane is preferably from 0.1% to 1.0%, and more preferably from 0.2% to 0.6%. When the concentration of the collagen sol is the lower limit described above or more, gelation is not excessively weak, while, when the concentration of the collagen sol is the upper limit described above or less, it is possible to obtain a porous membrane formed of a uniform collagen gel (in particular, a semipermeable membrane).

Furthermore, the obtained hydrogel may be dried to obtain dried hydrogel. Drying the hydrogel completely removes free water in the hydrogel and makes it possible to further advance the partial removal of bound water.

Furthermore, the obtained dried hydrogel may be rehydrated with PBS, the culture medium to be used, or the like to obtain Vitrigel (registered trademark).

The longer the period of the vitrification step (the step of advancing the partial removal of the bound water after completely removing the free water in the hydrogel), the more it is possible to obtain Vitrigel (registered trademark) with excellent transparency and strength when rehydrated. As necessary, it is possible to wash Vitrigel (registered trademark) obtained by rehydration after vitrification for a short period of time with PBS or the like and carry out re-vitrification.

As a drying method, it is possible to use various methods such as air drying, drying in a sealed container (circulating the air in the container and constantly supplying dry air), and drying in an environment where silica gel is placed. For example, as the method for air drying, it is possible to illustrate methods such as drying for 2 days in an incubator kept sterile at 10°C and a humidity of 40% or drying for a whole day and night in a clean bench in a sterile state at room temperature.

In the present specification, "Vitrigel (registered trademark)" refers to a gel in a stable state obtained by rehydration after vitrification of a traditional hydrogel and named by the present inventors as "Vitrigel (registered trademark)".

In addition, in the present specification, when describing in detail the step of manufacturing the porous membrane formed of hydrogel, the dried hydrogel immediately after the vitrification step without having undergone the rehydration step is simply referred to as "dried hydrogel". Here, the gel obtained through the rehydration step after the vitrification step is distinguished and represented as "Vitrigel (registered trademark)". In addition, the dried product obtained by vitrifying the Vitrigel (registered trademark) is referred to as "dried Vitrigel (registered trademark)". Further, the product obtained by carrying out the step of irradiating the dried Vitrigel (registered trademark) with ultraviolet rays is referred to as "dried Vitrigel (registered trademark) subjected to an ultraviolet irradiation treatment". In addition, a gel obtained by carrying out a rehydration step on the "dried Vitrigel (registered trademark) subjected to an ultraviolet irradiation treatment" is referred to as "Vitrigel (registered trademark) material". In addition, the dried product obtained by vitrifying the Vitrigel (registered trademark) material is referred to as "dried Vitrigel (registered trademark) material". Accordingly, "Vitrigel (registered trademark)" and "Vitrigel (registered trademark) material" are hydrates.

That is, the obtained Vitrigel (registered trademark) may be re-vitrified by re-drying to obtain dried Vitrigel (registered trademark).

Examples of drying methods include the same methods as shown above.

In addition, the obtained dried Vitrigel (registered trademark) may be irradiated with ultraviolet rays to obtain the "dried Vitrigel (registered trademark) subjected to an ultraviolet irradiation treatment".

It is possible to use a known ultraviolet irradiation device for the irradiation of the ultraviolet rays.

For the irradiation energy of ultraviolet rays to the dried Vitrigel (registered trademark), the total irradiation amount per unit area is preferably 0.1 mJ/cm² or more and 6000 mJ/cm² or less, more preferably 10 mJ/cm² or more and 4000 mJ/cm² or less, and even more preferably 100 mJ/cm² or more and 3000 mJ/cm² or less. When the total irradiation amount is in the above range, it is possible to make the transparency and strength of the Vitrigel (registered trademark) material obtained in the subsequent rehydration step particularly preferable.

In addition, the irradiation of the dried Vitrigel (registered trademark) with ultraviolet rays may be repeated a plurality of times. In a case where the irradiation of the dried Vitrigel (registered trademark) with ultraviolet rays is repeated, it is preferable to perform a step of rehydrating and re-vitrifying the dried Vitrigel (registered trademark) subjected to an ultraviolet irradiation treatment after the first irradiation with ultraviolet rays and then perform ultraviolet irradiation on the dried Vitrigel (registered trademark) material after the second or subsequent re-vitrification.

When the total irradiation amount of ultraviolet rays per unit area is the same, the irradiation of the dried Vitrigel (registered trademark) with ultraviolet rays is divided into a plurality of times and repeated, such that it is possible to increase the transparency and strength of the Vitrigel (registered trademark) material obtained in the subsequent rehydration step. In addition, the larger the number of divisions, the more preferable. For example, when the total irradiation amount per unit area of the irradiation of the dried Vitrigel (registered trademark) with the ultraviolet rays is in the range of 1000 mJ/cm² or more and 4000 mJ/cm² or less, the number of times of irradiation within the range is preferably 2 times or more and 10 times or less and more preferably 2 times or more and 6 times or less.

In addition, in a case where the irradiation of the dried Vitrigel (registered trademark) with ultraviolet rays is repeated, the portion irradiated with the ultraviolet rays may be divided into one surface and the other surface (upper surface and lower surface) of the dried Vitrigel (registered trademark) and irradiated, such that the total irradiation amount is set as the total irradiation amount of the ultraviolet rays per unit area on the dried Vitrigel (registered trademark).

It is considered that the increase in the strength and transparency of the Vitrigel (registered trademark) material obtained in the subsequent rehydration step from irradiating the dried Vitrigel (registered trademark) with ultraviolet rays is because the polymer compounds in the Vitrigel (registered trademark) material are cross-linked by the ultraviolet rays. That is, it is considered that the operation makes it possible to maintain high transparency and strength in the Vitrigel (registered trademark) material.

Furthermore, Vitrigel (registered trademark) material may be obtained by rehydrating the obtained dried Vitrigel (registered trademark) subjected to an ultraviolet irradiation treatment with PBS or the culture medium to be used.

Furthermore, a dried Vitrigel (registered trademark) material may be obtained by drying the obtained Vitrigel (registered trademark) material and carrying out re-vitrification.

Examples of drying methods include the same methods as shown above.

### [Frame Member]

In the cell encapsulation device, it is possible for a member forming a portion other than the porous membrane, that is, the frame member, to have liquid tightness.

In addition, the frame member may be air-permeable or non-air-permeable.

In a case where the frame member is air-permeable, the oxygen permeability coefficient may be, for example, 100 cm³/m² 24 hr atm or more and 5000 cm³/m² 24 hr atm or less, 1000 cm³/m² 24 hr atm or more and 3000 cm³/m² 24 hr atm or less, and 1200 cm³/m² 24 hr atm or more and 2500 cm³/m² 24 hr atm or less. Further, the carbon dioxide permeability coefficient may be, for example, 1000 cm³/m² 24 hr atm or more and 20000 cm³/m² 24 hr atm or less, 3000 cm³/m² 24 hr atm or more and 15000 cm³/m² 24 hr atm or less, 5000 cm³/m² 24 hr atm or more and 10000 cm³/m² 24 hr atm or less.

In addition, in a case where the frame member is not air-permeable, the oxygen permeability coefficient may be, for example, 100 cm³/m² 24 hr atm or less, or 50 cm³/m² 24 hr atm or less. Furthermore, the carbon dioxide permeability coefficient may be, for example, 1000 cm³/m² 24 hr atm or less, or 500 cm³/m² 24 hr atm or less.

In the cell encapsulation device, the material of the frame member may be any material suitable for culturing cells. Examples of the material forming the frame member include a metal material, a glass material, an elastomer material, a plastic including a dendritic polymer, a copolymer, and the like, without being limited thereto.

Examples of the metal material include stainless steel and the like.

Examples of the glass material include soda-lime glass, Pyrex (registered trademark) glass, Vycor (registered trademark) glass, quartz glass, and the like.

Examples of the elastomer material include urethane rubber, nitrile rubber, silicone rubber, silicone resin (for example, polydimethylsiloxane), fluorine rubber, acrylic rubber, isoprene rubber, ethylene propylene rubber, chlorosulfonated polyethylene rubber, epichlorohydrin rubber, chloroprene rubber, styrene-butadiene rubber, butadiene rubber, polyisobutylene rubber, and the like.

Examples of the dendritic polymer include poly(vinyl chloride), poly(vinyl alcohol), poly(methyl methacrylate), poly(vinyl acetate-co-maleic anhydride), poly(dimethylsiloxane) monomethacrylate, cyclic olefin polymer, fluorocarbon polymers, polystyrene, polypropylene, polyethyleneimine, and the like.

Examples of the copolymer include poly(vinyl acetate-co-maleic anhydride), poly(styrene-co-maleic anhydride), poly(ethylene-co-acrylic acid), derivatives thereof, and the like.

The material of the frame member may be formed of one type of the materials shown above, or may be formed of two types or more.

In addition, in a case where the frame member is formed of two types or more of the materials shown above, the frame member may be formed of a mixture of the materials shown above. Alternatively, the frame members may be formed by combining frame members formed of one type of material, and the materials forming each frame member may be different from each other.

In particular, the material of the frame member preferably includes an elastomer material, more preferably includes a silicone resin, and even more preferably includes polydimethylsiloxane.

In addition, the frame member is preferably formed of only an elastomer material or a combination of a plastic including a dendritic polymer and an elastomer material. In a case where the frame member is formed of a combination of a plastic and an elastomer material, it is preferable that the portion into which the suction tube is inserted is formed of an elastomer material and the other portions are formed of a plastic including a dendritic polymer. With the above configuration, it is possible to easily manufacture the cell encapsulation device with a suction tube of the present embodiment.

In addition, it is possible to appropriately select the shape of the frame member according to the overall shape of the cell encapsulation device and the parts forming the cell encapsulation device.

In addition, the frame member may be provided with a way to identify each cell encapsulation device (for example, coloring, printing, and the like).

### (Method for Manufacturing Frame Members)

It is possible to manufacture the frame member by a known method according to the material to be used.

Examples of manufacturing method in a case of using an elastomer material or a plastic as the material of the frame member include a compression molding method, an injection molding method, an extrusion molding method, and the like without being limited thereto.

In addition, examples of a manufacturing method in a case where a glass material is used as the material of the frame member include a droplet molding method, the Danner method, an overflow method, a float method, a blow molding method, a press molding method, and the like, without being limited thereto.

### [Adhesive Layer]

It is possible to set the thickness of each adhesive layer to, for example, 0.1 µm or more and 1000 µm or less.

Here, "the thickness of each adhesive layer" means the thickness of each and every adhesive layer, for example, the thickness of the first adhesive layer formed of a plurality of layers means the total thickness of all the layers forming the first adhesive layer.

The material for the adhesive layer may be any material which does not have cytotoxicity. In addition, it is possible to appropriately select the material of the adhesive layer according to each material of the porous membrane, the frame member, and the film having air tightness which are to be adhered.

In addition, it is possible to detachably attach the film having air tightness with respect to the outer surface of the porous membrane using an "adhesive having a low adhesiveness to a film having air tightness and a high adhesiveness to a porous membrane" or "an adhesive having a high adhesiveness to a film having air tightness and a low adhesiveness to a porous membrane".

For example, in a case where the porous membrane is a membrane filter and the film having air tightness is a silicon-coated polyethylene terephthalate (PET) film, it is possible for the silicon-coated PET film which is the film having air tightness to be detachably attached to the membrane filter using double-sided tape (Nitto Denko Corporation, No. 57210B, Lot No. 8328702, thickness: 0.1 mm) of a thin, foam body-based waterproof material for adhering the membrane filter and the silicon-coated surface of the PET film. In such a case, when the silicon-coated PET film is attached and detached, the adhesive of the double-sided tape of a thin, foam body-based waterproof material is exposed on the outer surface of the membrane filter. In the Examples described below, a use example is shown in which the adhesive of the double-sided tape of thin, foam body-based waterproof material is not exposed to the outer surface of the membrane filter even when the PET film is attached and detached.

Specific examples of the material of the adhesive layer include synthetic compound adhesives, natural compound adhesives, double-sided tape, and the like, without being limited thereto.

Examples of the synthetic compound adhesive include a urethane adhesive, a cyanoacrylate adhesive, polymethyl methacrylate (PMMA), a calcium phosphate adhesive, a resin cement, and the like.

Examples of the natural compound adhesive include fibrin glue, gelatin glue, and the like.

As the double-sided tape, tape used in medical applications and the like is suitably used.

Examples of the double-sided tape include tape having a structure in which an adhesive is laminated on both sides of a support tape, and the like.

Examples of the adhesive include known adhesives such as rubber-based, acrylic-based, urethane-based, silicone-based, and vinyl ether-based adhesives.

More specific examples of the double-sided tape include a double-sided tape for skin attachment manufactured by 3M Japan (product numbers: 1510, 1504XL, 1524, and the like), a double-sided adhesive tape for skin manufactured by Nitto Denko Corporation (product numbers: ST502, ST534) and the like), double-sided tape for medical use manufactured by Nichiban Medical Corp. (product numbers: #1088, #1022, #1010, #809SP, #414125, #1010R, #1088R, #8810R, #2110R, and the like), double-sided adhesive tape of a thin, foam body-based material manufactured by DIC (product numbers: #84010, #84015, #84020, and the like) and the like.

Here, in a case where the porous membrane 10a is transparent or translucent, it is possible to easily visually distinguish the top surface side and the bottom surface side, by using double-sided adhesive tapes of different colors such as white and black (for example, #84010WHITE and #84010BLACK manufactured by DIC, or the like) on the top surface and bottom surface of the respective frame members.

The adhesive layer may be formed of one type of the materials shown above, or may be formed of two types or more.

In addition, in a case where the adhesive layer is formed of two types or more of the materials shown above, the adhesive layer may be formed of a mixture of the materials shown above. Alternatively, the adhesive layer may be formed by laminating two or more adhesive layers formed of one type of material and the materials forming each adhesive layer may be different from each other.

### [Film having Air tightness]

It is possible to set the thickness of the film having air tightness to, for example, 10 µm or more and 1000 µm or less, 30 µm or more and 500 µm or less, or 50 µm or more and 200 µm or less.

Here, "the thickness of the film having air tightness" means the thickness of the entire film having air tightness, for example, the thickness of the film having air tightness having a plurality of layers means the total thickness of all the layers forming the film having air tightness.

As the material of the film having air tightness, any material having air tightness may be used.

Specifically, the material of the film having air tightness may be an organic material or an inorganic material.

Examples of the organic material include polyamide (for example, nylon), polyolefin-based resin, polyester-based resin, polystyrene-based resin, polycarbonate, polyamide-based resin, silicone resin, and the like, without being particularly limited thereto.

Examples of the inorganic material include ceramics, glass, and the like, without being particularly limited thereto.

The film having air tightness may be formed of one type of the materials shown above, or may be formed of two types or more.

In addition, in a case where the film having air tightness is formed of two types or more of the materials shown above, the film having air tightness may be formed of a mixture of the materials shown above. Alternatively, the film having air tightness may be formed by laminating two layers or more of films having air tightness formed of one type of material, and the materials forming each of the films having air tightness may be different from each other.

### (Method for Manufacturing Film Having Air tightness)

It is possible to manufacture the film having air tightness using a known method according to the material to be used.

Examples of a manufacturing method in a case where an organic material is used as the material of the film having air tightness include a compression molding method, a calendar molding method, an injection molding method, an extrusion molding method, inflation molding, and the like, without being limited thereto.

In addition, examples of a manufacturing method in a case of using glass as the material of the film having air tightness include the same method as the method shown in the above description (Method for Manufacturing Member).

In addition, examples of a manufacturing method in a case where a ceramic is used as a material of the film having air tightness include a dry forming method (for example, a mold forming method, a cold isostatic pressing method, a hot pressing method, a hot isostatic pressing method, and the like), plastic molding method (for example, a potter's wheel molding method, an extrusion molding method, an injection molding method), a cast molding method (for example, a slurry casting method, a pressure casting method, a rotary casting method, and the like), a tape molding method, and the like, without being limited thereto.

### [Support]

The material of the support may be an organic material or an inorganic material. Examples of the organic material and the inorganic material include the same materials as shown in the "Film having Air tightness" described above.

In addition, the shape of the support is, for example, a sheet shape, a rod shape, or the like, without being limited thereto.

In addition, the support may be provided with a way to identify each cell encapsulation device (for example, coloring, printing, and the like).

### (Method for Manufacturing Support)

It is possible to manufacture the support by a known method according to the material to be used. Specific examples of the manufacturing method include the same examples as shown in the "Method for Manufacturing Film Having Air tightness" described above.

### <Suction Tube>

The suction tube has a first tubular member and a second tubular member. Both end portions of the first tubular member and the second tubular member are open.

In addition, one end portion of the first tubular member and the second tubular member communicate with the first through hole and the second through hole of the frame member, respectively.

In addition, the other end portion of the first tubular member has a connection mechanism with a suction means.

Examples of the suction means include a pipettor, a syringe, a dropper, and the like.

Examples of the connection mechanism in a case where the suction means is a pipettor include a mechanism in which the shape of the inside of the other end portion of the first tubular member matches the shape of the tip portion of the pipettor, or the like. Due to this, it is possible to connect the cell encapsulation device with a suction tube of the present embodiment to the tip portion of the pipettor using the other end portion of the first tubular member.

In addition, the connection mechanism may have a filter in order to prevent foreign matter from entering the suction means. Examples of the filter include a known filter provided in an aerosol resistant tip and a hyper filter tip.

The material of the suction tube is not particularly limited and, for example, in a case where a cell encapsulation device is used as a medical cell transplantation device, the material of the suction tube is preferably a material having biocompatibility. Examples of the biocompatible material include the natural polymer compounds and the synthetic polymer compounds shown in the "Porous Membrane" described above.

In addition, in a case where the cell encapsulation device is used for constructing a multicellular structure used in an in vitro test system such as a test for assaying the activity of cells, the device may be a material having the biocompatibility described above, or may be a material suitable for culturing cells. Examples of the biocompatible material include the natural polymer compounds and the synthetic polymer compounds shown in the "Porous Membrane" described above. Materials suitable for culturing the cells include the same materials as shown in the "Member" described above.

In addition, more specific examples suitably used as the suction tube include a gel-loading tip, a siliconized tip, and the like.

### (Method for Manufacturing Suction Tube)

It is possible to manufacture the suction tube using a known method according to the material to be used.

As a specific manufacturing method, a tubular shape may be formed by using the same method as the method shown in the "Method for Manufacturing Porous Membrane" and "Method for Manufacturing Member" described above.

### <Method for Manufacturing Cell Encapsulation Device with Suction Tube>

It is possible to manufacture the cell encapsulation device of the present embodiment by assembling the porous membrane, the frame member, and the suction tube into a desired shape. In addition, a support and a film having air tightness may be provided as necessary.

The manufacturing methods of each of the porous membrane, the frame member, the support, the film having air tightness, and the suction tube are as described above.

More specifically, a detailed description will be given below of a method for manufacturing the cell encapsulation device with a suction tube of the present embodiment shown in Fig. 2C.

First, the frame member 10b having a desired size is prepared. Next, a first through hole and a second through hole are formed on the outer circumferential surface of the frame member 10b using an injection needle or the like, and the first tubular member 20a and the second tubular member 20b are respectively inserted therein. Next, the first adhesive layer 11a and the second adhesive layer 11b are formed on the top surface and bottom surface of the frame member 10b respectively, using an adhesive or a double-sided tape.

Next, two porous membranes (10a-1 and 10a-2) having the same size as the top surface and bottom surface of the frame member 10b, or being slightly larger than the top surface and bottom surface of the frame member 10b, are prepared. At this time, the two porous membranes (10a-1 and 10a-2) may be formed of the same material as each other or may be formed of different materials.

Next, the two porous membranes (10a-1 and 10a-2) are adhered via the first adhesive layer 11a and the second adhesive layer 11b so as to be the top surface and bottom surface of the member 10b, respectively.

Next, two films having air tightness (12a and 12b) having the same size as the porous membranes (10a-1 and 10a-2), or being slightly larger than the porous membranes (10a-1 and 10a-2), are prepared. At this time, the two films having air tightness (12a and 12b) may be formed of the same material as each other or may be formed of different materials.

Next, the third adhesive layer 11c and the fourth adhesive layer 11d are respectively formed on the prepared two films having air tightness (12a and 12b), using an adhesive or a double-sided tape.

Next, two films having air tightness (12a and 12b) are adhered to the porous membranes (10a-1 and 10a-2) attached to the top surface and bottom surface, via the third adhesive layer 11c and the fourth adhesive layer 11d respectively.

Next, as necessary, a specific sterilization treatment with ultraviolet irradiation or the like or a total sterilization treatment with γ-ray irradiation or the like is carried out and the sizes of the porous membrane 10a, the frame member 10b, the first tubular member 20a, or the second tubular member 20b are adjusted to make it possible to obtain the cell encapsulation device with a suction tube 400.

In addition, in a case where the cell encapsulation device is provided with a support, the support may be joined to the frame member 10b in advance. Alternatively, a support may be joined to the assembled cell encapsulation device 10. As the joining method, fixing may be carried out by the same method as the joining method of the porous membrane and the frame member described above, or attachment may be carried out by using a fastener or the like so as to be detachable.

### <Set for Cell Encapsulation Device with Suction Tube>

A set for a cell encapsulation device with a suction tube of the present embodiment is provided with the cell encapsulation device with a suction tube of the embodiment described above, and a rack.

According to the set for a cell encapsulation device with a suction tube of the present embodiment, it is possible to efficiently perform continuous production of cell encapsulation devices.

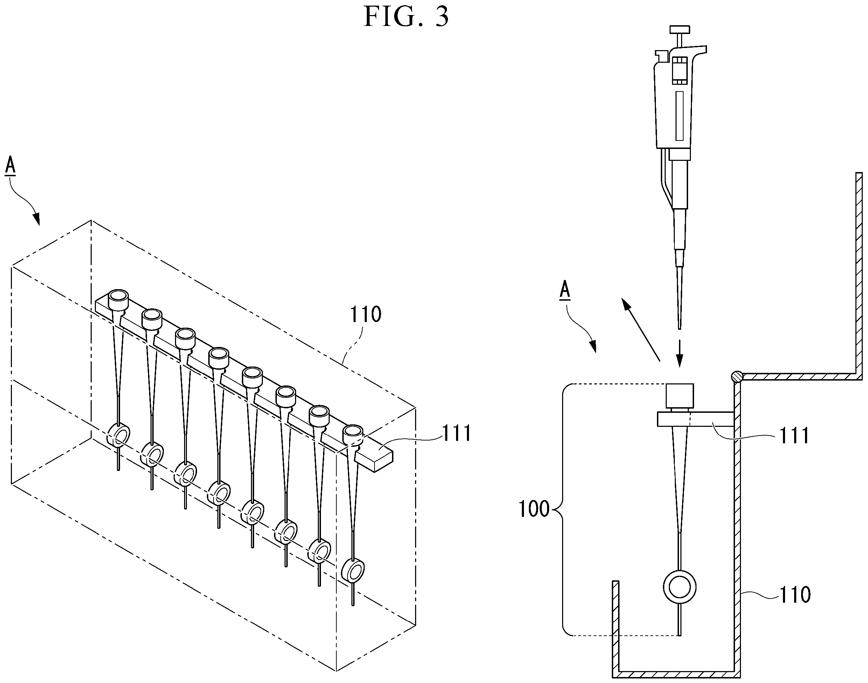

Fig. 3 is a perspective view and a side view showing an example of the set for a cell encapsulation device with a suction tube of the present embodiment. A detailed description will be given of the structure of the set for a cell encapsulation device with a suction tube of the present embodiment with reference to Fig. 3.

A set A for a cell encapsulation device with a suction tube shown in Fig. 3 is accommodated in a state in which a plurality of cell encapsulation devices with a suction tube 100 are hooked and locked on U-shaped locking portions 111 of a rack 110. In addition, for example, in a case where the lid of the rack 110 is opened and closed as shown in the side view of Fig. 3, it is possible to connect with the suction means such as a pipettor while accommodating the cell encapsulation device with a suction tube in the rack 110. Further, pulling the cell encapsulation device with a suction tube 100 obliquely upward in the front direction while being connected to the suction means makes it possible to take out the porous membrane of the cell encapsulation device without damage. Therefore, even in continuous production of cell encapsulation devices, it is possible to efficiently take out and use the cell encapsulation device with a suction tube.

In addition, it is possible to perform total sterilization of the set for a cell encapsulation device with a suction tube of the present embodiment with γ-rays or the like in a state where a plurality of the cell encapsulation devices with a suction tube 100 are accommodated in a rack.

### <Rack>

The rack 110 has a locking portion 111 for accommodating the cell encapsulation device with a suction tube 100. The locking portion 111 may be U-shaped as shown in Fig. 3, or may have another shape. In particular, the shape of the locking portion 111 is preferably a U-shape, since it is possible to take out the cell encapsulation device with a suction tube 100 without damaging the porous membrane.

As the rack, it is possible to use the same material as the material of known racks which accommodate normal pipette tips or the like.

### <Method for Encapsulating Cells>

The method for encapsulating cells of the present embodiment is a method using the cell encapsulation device with a suction tube of the embodiments described above.

According to the encapsulation method of the present embodiment, it is possible to easily encapsulate cells in the device.

In the encapsulation method of the present embodiment, first, the suction means is connected to the cell encapsulation device with the suction tube via the connection mechanism of the first tubular member to suction the suspension of cells in the cell encapsulation device with a suction tube of the embodiment described above.

Examples of the suction means include a pipettor, a syringe, a dropper, and the like.

Examples of cells to be encapsulated include vertebrate cells such as mammalian cells, avian cells, reptile cells, amphibian cells, and fish cells; invertebrate cells such as insect cells, crustacean cells, molluscan cells, and protozoan cells; bacteria such as gram-positive bacteria (for example, Bacillus species or the like) and gram-negative bacteria (for example, Escherichia coli or the like); yeast, plant cells, small living individuals formed of a single cell or a plurality of cells, and the like.

Examples of the small living individual include unicellular organisms, microcrustaria, planaria (including regenerated planaria after fine cutting), terrestrial arthropod larvae, nematodes, plant seeds (particularly germinated seeds), callus, protoplasts, marine microorganisms, larva fry, larvae, and the like, without being limited thereto. Examples of the unicellular organisms include amoeba, paramecium, closterium, pinnularia, chlorella, euglena, phacus, and the like. Examples of the microcrustaria include daphnia, artemia larvae, copepods, ostracoda, aphelaria larvae, phyllocarida shrimp larvae, peracarida shrimp larvae, and eucarida shrimp larvae, and the like. Examples of marine microorganisms include marine bacteria, algae, and the like. Examples of the marine bacteria include bacteria belonging to the genuses vibrio, pseudomonas, eromonas, alteromonas, flavobacterium, cytophaga, flexibacter, and the like. Examples of algae include cyanobacteria, cryptophytes, gonyaulax, diatom, raphidophytes, golden algae, haptophytes, euglenophytes, prasinophyceae, green algae, and the like.

For example, in a case where germinated seeds are cultured using the cell encapsulation device of the present embodiment, the top surface of the cell encapsulation device has a hardness such that it is possible for germinated sprouts to penetrate through and the cell encapsulation device is formed of a biodegradable material and, due to this, it is possible to directly plant a device, into which germinated seeds are put, in soil to grow a plant. In the present specification, the term "biodegradable material" means a material having a property of being decomposed into inorganic substances by microorganisms or the like in soil or water.

Examples of vertebrate cells (in particular, mammalian cells) include reproductive cells (sperm, ovum, and the like), somatic cells, stem cells, and precursor cells forming a living body, cancer cells separated from a living body, cells (cell lines) separated from a living body and stably maintained outside by being immortalized, cells separated from a living body and artificially genetically modified, cells separated from a living body and with the nuclei artificially exchanged, and the like, without being limited thereto. In addition, a cell mass (spheroid) of these cells may be used. In addition, a small tissue piece separated from normal tissue or cancer tissue of a living body may be used as it is in the same manner as a mass of cells.

Examples of somatic cells forming a living body include cells or the like collected from any tissue such as skin, kidney, spleen, adrenal gland, liver, lung, ovary, pancreas, uterus, stomach, colon, small intestine, large intestine, bladder, prostate, testis, thymus, muscle, connective tissue, bone, cartilage, vascular tissue, blood, heart, eye, brain, and nerve tissue, without being limited thereto. More specific examples of the somatic cells include fibroblasts, bone marrow cells, immune cells (for example, B lymphocytes, T lymphocytes, neutrophils, macrophages, monocytes, and the like), red blood cells, platelets, osteocytes, bone marrow cells, pericytes, dendritic cells, epidermal keratinocytes (keratinocytes), adipocytes, mesenchymal cells, epithelial cells, epidermal cells, endothelial cells, vascular endothelial cells, lymphatic endothelial cells, hepatocytes, islet cells (for example, α cells, β cells, δ cells, ε cells, PP cells, and the like), chondrocytes, cumulus cells, glial cells, nerve cells (neurons), oligodendrocytes, microglia, astrocytes, cardiomyocytes, esophageal cells, muscle cells (for example, smooth muscle cells, skeletal muscle cells, and the like), melanocytes, mononuclear cells, and the like, without being limited thereto.

A stem cell is a cell which combines the ability to replicate itself and the ability to differentiate into cells of a plurality of other lines. Examples of stem cells include embryonic stem cells (ES cells), embryonic tumor cells, embryonic reproductive stem cells, induced pluripotent stem cells (iPS cells), neural stem cells, hematopoietic stem cells, mesenchymal stem cells, hepatic stem cells, pancreatic stem cells, muscle stem cells, reproductive stem cells, intestinal stem cells, cancer stem cells, hair follicle stem cells, and the like, without being limited thereto.

A precursor cell is a cell in the stage of being differentiated from the stem cell into a specific somatic cell or reproductive cell.

A cancer cell is a cell derived from a somatic cell and acquiring infinite proliferative capacity and is a malignant neoplasm which invades or causes metastasis in the surrounding tissue. Examples of cancers from which cancer cells are derived include breast cancer (for example, invasive breast ductal cancer, non-invasive ductal cancer, inflammatory breast cancer, and the like), prostate cancer (for example, hormone-dependent prostate cancer, hormone-independent prostate cancer, and the like), pancreatic cancer (for example, pancreatic duct cancer, and the like), stomach cancer (for example, papillary adenocarcinoma, mucinous adenocarcinoma, adenosquamous cancer, and the like), lung cancer (for example, non-small cell lung cancer, small cell lung cancer, malignant mesothelioma, and the like), colon cancer (for example, gastrointestinal stromal tumors, and the like), rectal cancer (for example, gastrointestinal stromal tumors, and the like), colorectal cancer (for example, familial colorectal cancer, hereditary non-polyposis colon cancer, gastrointestinal stromal tumors, and the like), small bowel cancer (for example, non-Hodgkin's lymphoma, gastrointestinal stromal tumors, and the like), esophageal cancer, duodenal cancer, tongue cancer, pharyngeal cancer (for example, nasopharyngeal cancer, oropharyngeal cancer, hypopharyngeal cancer, and the like), head and neck cancer, salivary gland cancer, brain tumors (for example, pineal gland astrocytoma, pilocytic astrocytoma, diffuse astrocytoma, anaplastic astrocytoma, and the like), neurinomas, liver cancer (for example, primary liver cancer, extrahepatic bile duct cancer, and the like), kidney cancer (for example, renal cell cancer, transitional epithelial cancer of renal pelvis and ureter, and the like), gallbladder cancer, pancreatic cancer, endometrial cancer, cervical cancer, ovarian cancer (for example, epithelial ovarian cancer, extragonadal germ cell tumors, ovarian germ cell tumors, low grade ovarian tumors, and the like), bladder cancer, urethral cancer, skin cancer (for example, intraocular (ocular) melanoma, Merkel cell cancer, and the like), angioma, malignant lymphoma (for example, reticulosarcoma, lymphosarcoma, Hodgkin's disease, and the like), melanoma (malignant melanoma), thyroid cancer (for example, medullary cancer of the thyroid, and the like), parathyroid cancer, nasal cancer, paranasal sinus cancer, bone tumors (for example, osteosarcoma, Ewing's tumor, uterine sarcoma, soft tissue sarcoma, and the like), metastatic medulloblastoma, angiofibroma, protruding dermal fibrosarcoma, retinal sarcoma, penile cancer, testicular tumors, pediatric solid cancer (for example, Wilms tumor, pediatric renal tumors, and the like), Kaposi's sarcoma, Kaposi's sarcoma caused by AIDS, maxillary sinus tumors, fibrous histiocytoma, leiomyosarcoma, rhabdomyosarcoma, chronic myeloproliferative disease, leukemia (for example, acute myelogenous leukemia, acute lymphoblastic leukemia, and the like) and the like, without being limited thereto.

In addition, in the present specification, the Chinese character for "cancer" is used to indicate a diagnosis name and the Japanese characters for "cancer" are used to represent a generic term for a malignant neoplasm.

A cell line is a cell which acquired infinite proliferative capacity due to artificial manipulation in vitro. Examples of cell lines include HCT116, Huh7, HEK293 (human embryonic kidney cells), HeLa (human cervical cancer cell line), HepG2 (human liver cancer cell line), UT7/TPO (human leukemia cell line), CHO (Chinese hamster ovary cell line), MDCK, MDBK, BHK, C-33A, HT-29, AE-1, 3D9, Ns 0/1, Jurkat, NIH3T3, PC12, S2, Sf9, Sf21, High Five, Vero, and the like, without being limited thereto.

In addition, it is preferable that the cells are suctioned and encapsulated in a state of a suspension of cells. In a case where the cells are animal cells, the culture medium used for the suspension of cells may be a basic culture medium including components (inorganic salts, carbohydrates, hormones, essential amino acids, non-essential amino acids, vitamins, and the like) necessary for cell survival and proliferation and it is possible to appropriately select the culture medium according to the type of cells. Examples of the culture medium include Dulbecco's Modified Eagle's Medium (DMEM), Minimum Essential Medium (MEM), RPMI-1640, Basal Medium Eagle (BME), Dulbecco's Modified Eagle's Medium: Nutrient Mixture F-12 (DMEM/F-12), Glasgow Minimum Essential Medium (Glasgow MEM), and the like, without being limited thereto.

In addition, a culture medium of a bacterium, a yeast, a plant cell, and a small living individual formed of a single cell or a plurality of cells thereof may be prepared as a culture medium having a composition suitable for the growth of each of the above.

In addition, in the method for encapsulating cells of the present embodiment, an extracellular matrix-derived component, a physiologically active substance, and the like may be mixed and injected into a culture medium in which cells are suspended.

Examples of the cell matrix-derived component include the same examples as shown in the "Porous Membrane" described above.

In addition, examples of physiologically active substances include cell proliferation factors, differentiation inducing factors, cell adhesion factors, and the like, without being limited thereto. For example, by including a differentiation inducing factor, in a case where the cells to be injected are stem cells, precursor cells, or the like, it is possible to induce differentiation of the stem cells or the precursor cells and to construct a multicellular structure reproducing a desired tissue.

In addition, in the encapsulation method of the present embodiment, a culture medium in which the cells are suspended may be injected so as to fill the capacity of the cell encapsulation device, or an amount less than the capacity of the cell encapsulation device may be injected.

It is possible to set the cell encapsulation device with the suction tube in which the cells are encapsulated as a cell encapsulation device in which the cells are encapsulated by removing the first tubular member and the second tubular member from the frame member.

It is possible to use the cell encapsulation device in which the obtained cells are encapsulated, for example, for culturing cells, transporting cells, tissue-type chips, organ-type chips, organ-type chip systems, and the like.

In the present specification, "tissue" refers to a unit of a structure gathered in a pattern based on a certain lineage in which one type of stem cell is differentiated, and which has a single role as a whole. For example, in epidermal keratinocytes, stem cells existing in the basal layer of the epidermis are differentiated into cells forming the granular layer through the spinous layer and are terminally differentiated to form a stratum corneum so as to exhibit a barrier function as the epidermis. Thus, constructing a multicellular structure including one type of cell derived from one cell lineage makes it possible for the tissue-type chip of the present embodiment to reproduce, for example, epithelial tissue, connective tissue, muscle tissue, nerve tissue, and the like.

In addition, in the present specification, an "organ" is formed of two or more types of tissues and has one function as a whole. Thus, constructing a multicellular structure including at least two types of cells having different cell lineages makes it possible for the organ-type chip of the present embodiment to reproduce, for example, a stomach, intestines, a liver, a kidney, and the like.

Furthermore, in the present specification, "organ system" refers to a group of two or more organs having similar functions and a group of two or more organs having a series of functions as a whole. Thus, by combining a plurality of tissue-type chips or organ-type chips, it is possible for the organ-type chip system of the present embodiment to reproduce, for example, organ systems such as a digestive system, a cardiovascular system, a respiratory system, a urinary system, a reproductive system, an endocrine system, a sensory organ system, a nervous system, an exerciser system, and a nervous system. Living bodies maintain homeostasis by interactions between these organ systems. In the organ-type chip system of the present embodiment, since it is possible to combine a plurality of organ-type chips of different organ systems, it is also possible to analyze the interaction between organs of different organ systems. For example, in an organ-type chip system in which a small intestine-type chip, a liver-type chip, and a neural-type chip are connected in this order, in a case where a drug is added to the small intestine-type chip, the drug absorbed by the small intestine-type chip is metabolized by the liver-type chip, and it is possible to analyze the toxicity and the like exerted by the liver metabolites of the drug discharged by the liver-type chip on the neural-type chip.

### EXAMPLES

A description will be given below of the present invention with reference to Examples; however, the present invention is not limited to the following Examples.

### [Production Example 1] Production of Cell Encapsulation Device with Suction Tube (Vitrigel (registered trademark)-silicon ring-Vitrigel (registered trademark))

### 1. Preparation of Silicon Ring

A silicon sheet (As One Corporation, thickness: 2 mm, 2-9318-01) was punched with a φ13 mm × φ8 mm punching blade (manufactured by Morishita Seihan Ltd.) to produce a silicon ring. Next, the silicon ring was sterilized by immersing in 70% ethanol for 10 minutes and washing with PBS 3 times.

Next, a hole was made in the sterilized silicon ring with a 24G injection needle (NN-2432R, 0.55 mm × 32 mm, manufactured by Terumo Corporation), and a gel-loading tip (As One Corporation, 2-4493-06) was inserted thereto. In the same manner, a hole was made on the 180-degree symmetrical side with a 24G injection needle and a gel-loading tip was inserted thereto.

Next, double-sided tape for adhering silicone rubber (Nitto Denko Corporation, No. 5302A, thickness: 0.085 mm) was punched out with a φ12 mm × φ8.5 mm punching blade (manufactured by Morishita Seihan Ltd.) and stuck on both surfaces of the silicon ring. At this time, silicon adhesive surface (release paper: transparent side) of the double-sided tape was attached to the silicon ring.

### 2. Preparation of Vitrigel (registered trademark)

Below, the description of "registered trademark" from "Vitrigel (registered trademark)" may be omitted. In a well (φ15 mm) of an acrylic 24-well plate (manufactured by Kishimoto Industry Co., Ltd.), a φ13 mm silicon-coated PET film (manufactured by Fujimori Kogyo Co., Ltd., thickness: 75 µm) support was placed with the silicon-coated surface facing up, 0.36 mL of 0.25% collagen sol obtained by mixing an equal amount of a bovine-derived native collagen solution (manufactured by Koken Co., Ltd., I-AC, collagen concentration of 0.5% by mass) and a culture medium was added dropwise thereon. Next, after gelation, vitrification, rehydration, and re-vitrification of the collagen sol, the silicon-coated PET film to which the dried collagen Vitrigel membrane was adsorbed was taken out from the well. This series of operations was performed twice to produce two dried collagen Vitrigel membranes with a φ13 mm silicon-coated PET film support.

### 3. Production of Cell Encapsulation Device with Suction Tube (Vitrigel-Silicon Ring-Vitrigel)

Next, the dried collagen Vitrigel membrane with the silicon-coated PET film support produced in "2." was stuck to the silicon ring produced in "1." to which the double-sided tape was stuck. In the same manner, a dried collagen Vitrigel membrane was stuck on the opposite surface of the silicon ring.

Next, the PET film was peeled off from the dried collagen Vitrigel membrane, one of the two gel-loading tips was cut off except for the tip portion, and a cell encapsulation device with a suction tube (Vitrigel-silicon ring-Vitrigel) was obtained.

### 4. Encapsulation of Culture medium

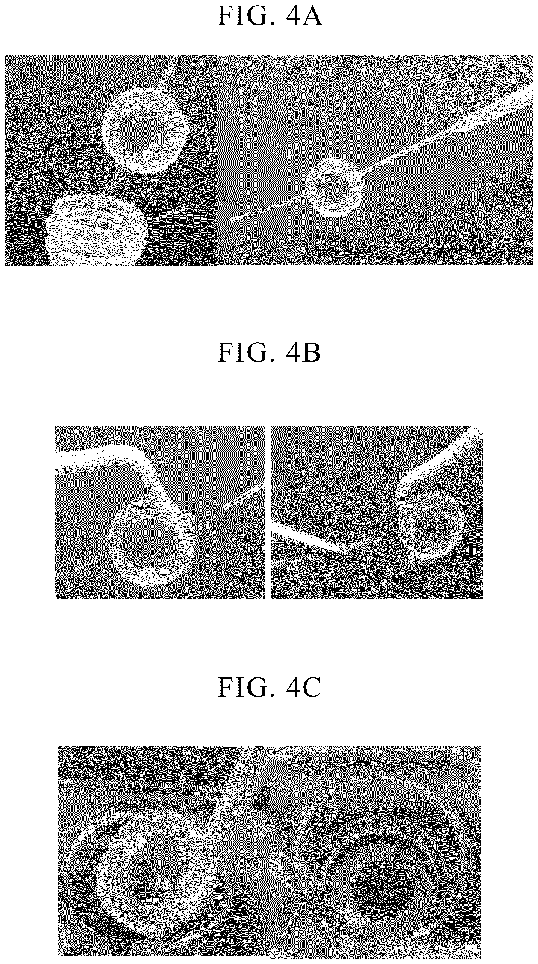

Next, a pipette was mounted on the obtained cell encapsulation device with a suction tube (Vitrigel-silicon ring-Vitrigel). Next, the tip of the gel-loading tip mounted on the pipette was made to touch and draw in the culture medium (refer to Fig. 4A).

Next, the gel-loading tip on the side mounted on the pipette was removed first. Next, the tip of the gel-loading tip on the opposite side was removed to obtain a cell encapsulation device (Vitrigel-silicon ring-Vitrigel) in which a culture medium was encapsulated (refer to Fig. 4B).

Next, the obtained cell encapsulation device (Vitrigel-silicon ring-Vitrigel) in which the culture medium was encapsulated was submerged in a 24-well plate including the culture medium in a well (refer to Fig. 4C).

From the above, it was confirmed that it was possible to easily encapsulate the culture medium in the cell encapsulation device (Vitrigel-silicon ring-Vitrigel). In addition, using a cell encapsulation device (Vitrigel-silicon ring-Vitrigel) also makes it possible to easily encapsulate a suspension of cells.

### [Production Example 2] Production of Cell Encapsulation Device (Vitrigel-Silicon Ring-Filter)

### 1. Preparation of Silicon Ring

First, a silicon ring was prepared using the same method as in "1." of Production Example 1.

### 2. Preparation of Vitrigel

Next, using the same method as in "2." of Production Example 1, one dried collagen Vitrigel membrane with a φ13 mm silicon-coated PET film (thickness: 75 µm) support was produced.

### 3. Preparation of Filter

Next, a filter (Omnipore (trademark) membrane (for both water-based and solvent-based), JHWP4700, pore size: 0.45 µm, diameter: φ47 mm) was punched at φ11 mm by a punching machine, sterilized by being immersed in 70% ethanol for 10 minutes, and dried.

### 4. Production of Cell Encapsulation Device with Suction Tube (Vitrigel-Silicon Ring-Filter)

Next, the dried collagen Vitrigel membrane with the silicon-coated PET film support produced in "2." was stuck to the silicon ring produced in "1." to which the double-sided tape was stuck.

Next, the filter produced in "3." was stuck to the surface on the opposite side to the surface to which the dried collagen Vitrigel membrane was attached.

Next, a silicon-coated PET film (manufactured by Fujimori Kogyo Co., Ltd., thickness: 75 µm) and a thin, foam body-based waterproof material double-sided tape (Nitto Denko Corporation, No. 57210B, thickness: 0.1 mm) were each punched one at a time with a φ12 mm x φ8.5 mm punching blade. The obtained ring-shaped silicon-coated PET film was sterilized with 70% ethanol. After drying, the non-silicon coated surface of the ring-shaped PET film and the ring-shaped thin, foam body-based waterproof material double-sided tape were stuck together.

Next, on the silicon ring on which Vitrigel is attached on one surface and the filter is attached on the other surface, the ring-shaped PET film was attached on the surface on which the filter is attached, such that the surface on which the double-sided tape was attached and the filter were in contact with each other.

Next, a silicon-coated PET film (manufactured by Fujimori Kogyo Co., Ltd., thickness: 75 µm) was punched with a φ16 mm punching blade and a thin foam base material waterproof double-sided tape (Nitto Denko Corporation, No. 57210B, thickness: 0.1 mm) was punched with a φ12 mm x φ8.5 mm punching blade. The obtained circular silicon-coated PET film was sterilized with 70% ethanol. After drying, the non-silicon-coated surface of the circular PET film and a ring-shaped thin, foam body-based waterproof material double-sided tape were stuck together. At this time, the double-sided tape was stuck as close to the center of the circular PET film as possible.

Next, for the silicon ring on which Vitrigel was attached on one surface and the filter was attached on the other surface, with the ring-shaped PET film attached to the filter, the silicon-coated surface of the ring-shaped PET film and the surface of the circular PET film, on which the double-sided tape was stuck, were attached to be in contact with each other.

Next, the PET film was peeled off from the dried collagen Vitrigel membrane, one of the two gel-loading tips was cut off leaving only the tip portion, and a cell encapsulation device with a suction tube (Vitrigel-silicon ring-filter) was obtained.

### 5. Encapsulation of Culture medium

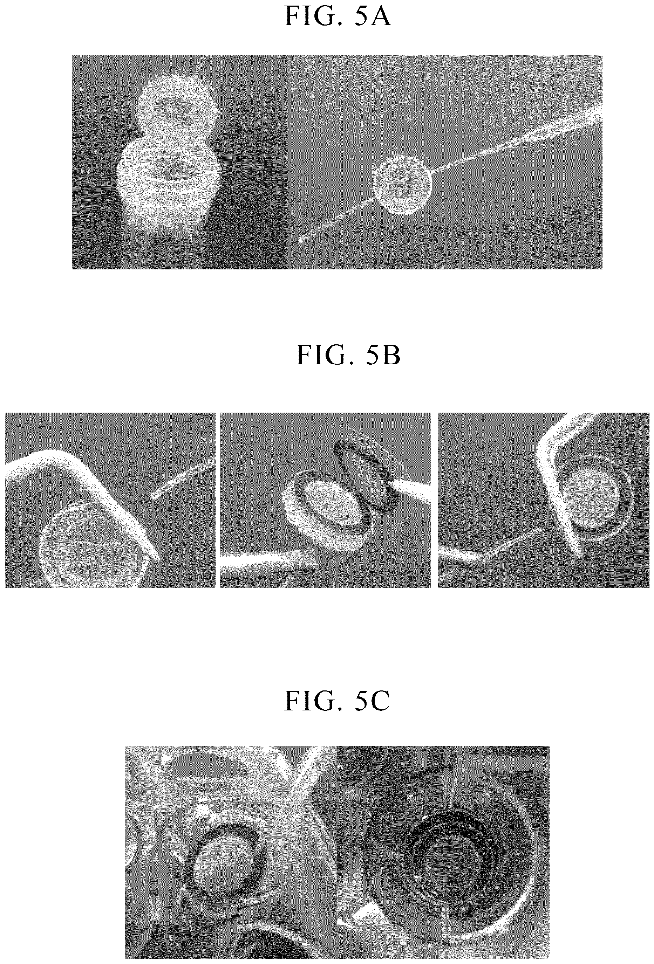

Next, a pipette was mounted on the obtained cell encapsulation device with a suction tube (Vitrigel-silicon ring-filter). Next, the tip of the gel-loading tip mounted on the pipette was made to touch and draw in the culture medium (refer to Fig. 5A).

Next, the gel-loading tip on the side mounted on the pipette was removed first. Next, the PET film stuck on the filter surface was peeled off. Next, the tip of the gel-loading tip on the opposite side was removed to obtain a cell encapsulation device (Vitrigel-silicon ring-filter) in which the culture medium was encapsulated (refer to Fig. 5B).

Next, the obtained cell encapsulation device (Vitrigel-silicon ring-filter) in which the culture medium was encapsulated was submerged in a 24-well plate including the culture medium in a well (refer to Fig. 5C).

From the above, it was confirmed that it was possible to easily encapsulate the culture medium in the cell encapsulation device (Vitrigel-silicon ring-filter). In addition, using the cell encapsulation device (Vitrigel-silicon ring-filter) also makes it possible to easily encapsulate a suspension of cells.

### [Production Example 3] Production of Cell Encapsulation Device (Filter-Silicon Ring-Filter)

### 1. Preparation of Silicon Ring

First, a silicon ring was prepared using the same method as in "1." of Production Example 1.

### 2. Preparation of Filter

Next, two filters (Omnipore (trademark) membrane (for both water-based and solvent-based), JHWP4700, pore size: 0.45 µm, diameter: φ47 mm) were punched out into two sheets at φ11 mm by a punching machine, sterilized by being immersed 70% ethanol for 10 minutes, and dried.

### 3. Production of Cell Encapsulation Device with Suction Tube (Filter-Silicon Ring-Filter)

Next, the filter produced in "2." was stuck to both surfaces of the silicon ring produced in "1." to which the double-sided tape was stuck.

Next, a silicon-coated PET film (manufactured by Fujimori Kogyo Co., Ltd., thickness: 75 µm) and a thin, foam body-based waterproof material double-sided tape (Nitto Denko Corporation, No. 57210B, thickness: 0.1 mm) were each punched with a φ12 mm x φ8.5 mm punching blade, two pieces at a time. The obtained ring-shaped, silicon-coated PET film was sterilized with 70% ethanol. After drying, two sets were produced in which the surface of a ring-shaped PET film which was not silicon-coated and a ring-shaped thin, foam body-based waterproof material double-sided tape were stuck together.

Next, for the silicon ring to which filters were attached on both surfaces, ring-shaped PET films were attached on the surfaces to which the filters were attached, such that the surfaces to which the double-sided tape was attached and the filters were in contact.

Next, a silicon-coated PET film (manufactured by Fujimori Kogyo Co., Ltd., thickness: 75 µm) was punched with a φ16 mm punching blade, and a thin, foam body-based waterproof material double-sided tape (Nitto Denko Corporation, No. 57210B, thickness: 0.1 mm) was punched out with a φ12 mm × φ8.5 mm punching blade, two pieces at a time. The obtained circular silicon-coated PET film was sterilized with 70% ethanol. After drying, two sets were produced in which the non-silicon coated surface of the circular PET film and the ring-shaped thin, foam body-based waterproof material double-sided tape were stuck together. At this time, the double-sided tape was stuck as close to the center of the circular PET film as possible.

Next, on one surface of the silicon ring to which filters were attached on both surfaces and ring-shaped PET films were attached on the filters, a silicon-coated surface of the ring-shaped PET film and the surface of the circular PET film to which double-sided tape was stuck were attached so as to be in contact.

Next, in the same manner, on the opposite surface of the silicon ring, the filter, the ring-shaped PET film, and the φ16 mm circular PET film were attached in this order.

Next, one of the two gel-loading tips was cut off leaving only the tip portion and a cell encapsulation device with a suction tube (filter-silicon ring-filter) was obtained.

### 4. Encapsulation of Culture medium

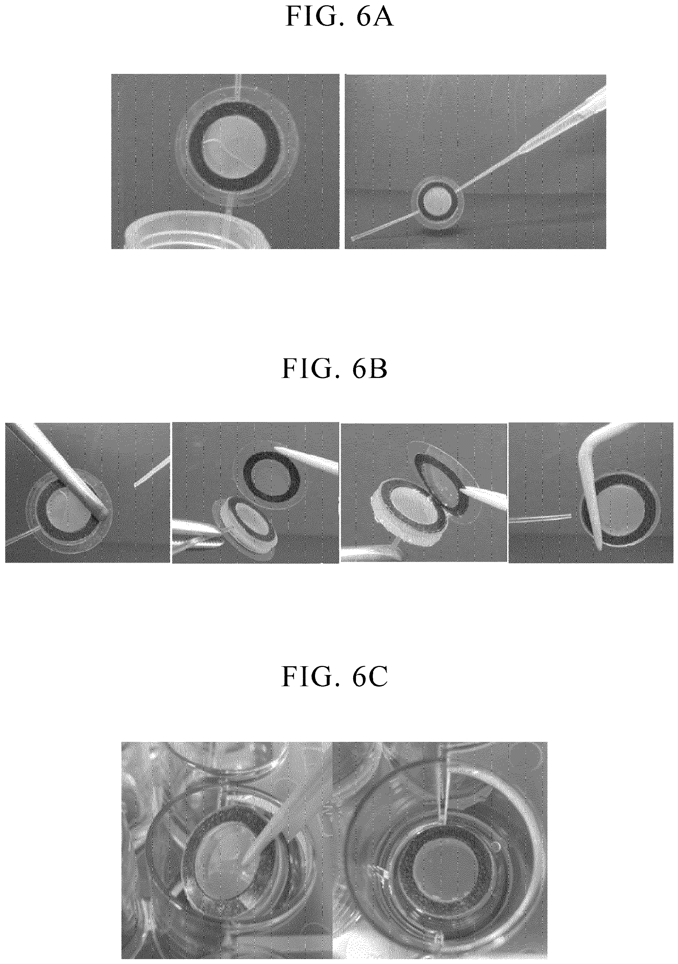

Next, a pipette was mounted on the obtained cell encapsulation device with a suction tube (filter-silicon ring-filter). Next, the tip of the gel-loading tip mounted on the pipette was made to touch and draw in the culture medium (refer to Fig. 6A).

Next, the gel-loading tip on the side mounted on the pipette was removed first. Next, the PET film stuck on the filter surface of both surfaces was peeled off. Next, the tip of the gel-loading tip on the opposite side was removed to obtain a cell encapsulation device (filter-silicon ring-filter) in which the culture medium was encapsulated (refer to Fig. 6B).

Next, the obtained cell encapsulation device (filter-silicon ring-filter) in which the culture medium was encapsulated was submerged in a 24-well plate including the culture medium in the wells (refer to Fig. 6C).

From the above, it was confirmed that it was possible to easily encapsulate the culture medium in the cell encapsulation device (filter-silicon ring-filter). In addition, using a cell encapsulation device (filter-silicon ring-filter) also makes it possible to easily encapsulate a suspension of cells in the same manner.

### [Production Example 4] Production of Cell Encapsulation Device (Filter-Plastic Ring-Filter)

A cell encapsulation device (filter-plastic ring-filter) was manufactured using the same method as in Production Example 3 except that a plastic ring was used instead of the silicon ring. Specifically, the cell encapsulation device was produced by sticking filters (Omnipore (trademark) membranes (for both water-based and solvent-based), JHWP4700, pore size: 0.45 µm) on both surfaces of a plastic ring (7.98 mm in inner diameter, 13.0 mm in outer diameter, and 2.0 mm in thickness) with wall hole paths able to be closed with a stainless steel ball as a stopper.

### [Example 1] Production of Spheroid

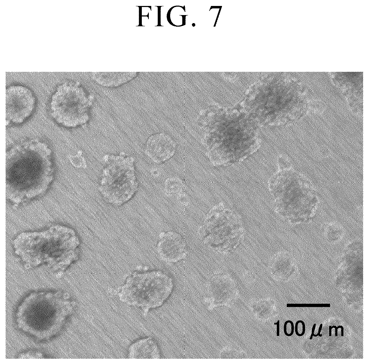

HepG2 cells (human liver cancer cell line) were encapsulated in the cell encapsulation device (filter-plastic ring-filter) obtained in Production Example 4 and cultured for 10 days. Due to this, it was confirmed that it is possible to produce spheroids of HepG2 cells (refer to Fig. 7).

### [Industrial Applicability]

According to the cell encapsulation device with a suction tube of the present embodiment, it is possible to easily encapsulate cells, the cell protection performance is excellent, handling is easy, and it is possible to culture cells for long periods.

### [Reference Signs List]

- 1a:: One end portion of first tubular member
- 1b:: Other end portion of first tubular member
- 2a:: One end portion of second tubular member
- 2b:: Other end portion of second tubular member
- 3:: Connection mechanism with suction means
- 10:: Cell encapsulation device
- 10a:: Porous membrane
- 10a-1:: Top surface side porous membrane
- 10a-2:: Bottom surface side porous membrane
- 10b:: Frame member
- 11a:: First adhesive layer
- 11b:: Second adhesive layer
- 11c:: Third adhesive layer
- 11d:: Fourth adhesive layer
- 12a:: Film having air tightness of top surface side
- 12b:: Film having air tightness of bottom surface side
- 20:: Suction tube
- 20a:: First tubular member
- 20b:: Second tubular member
- 100, 200, 300, 400:: Cell encapsulation device with suction TUBE
- 110:: Rack
- 111:: Locking portion
- A:: Set for cell encapsulation device with suction tube

## Claims

1. A cell encapsulation device with a suction tube comprising:
a cell encapsulation device for constructing a multicellular structure formed by culturing cells, the cell encapsulation device being provided with a porous membrane on at least one of a top surface and a bottom surface and a frame member at a side surface; and
a suction tube having a first tubular member and a second tubular member,
wherein a first through hole and a second through hole are formed in an outer circumferential surface of the frame member, the first through hole and the second through hole communicating with one end portion of the first tubular member and one end portion of the second tubular member, respectively,
the other end portion of the first tubular member has a connection mechanism to a suction means at the other end portion thereof, and
the other end portion of the second tubular member is open to the outside.

2. The cell encapsulation device with a suction tube according to claim 1,
wherein the first tubular member and the second tubular member are arranged to face each other, via the cell encapsulation device.

3. The cell encapsulation device with a suction tube according to claim 1 or 2,
wherein the cell encapsulation device injects multiple cells suspended in a culture medium and an internal volume is 10 mL or less.

4. The cell encapsulation device with a suction tube according to any one of claims 1 to 3,
wherein the porous membrane is a semipermeable membrane having liquid tightness in a gas phase and a semipermeable property in a liquid phase.

5. The cell encapsulation device with a suction tube according to claim 4,
wherein the semipermeable membrane includes a material having biocompatibility.

6. The cell encapsulation device with a suction tube according to claim 5,
wherein the material having biocompatibility is a gelling component derived from extracellular matrices.

7. The cell encapsulation device with a suction tube according to claim 6,
wherein the gelling component derived from extracellular matrices is native collagen or atelocollagen.

8. The cell encapsulation device with a suction tube according to any one of claims 1 to 7,
wherein a film having air tightness is further attachable to and detachable from an outer surface of the porous membrane.

9. The cell encapsulation device with a suction tube according to any one of claims 1 to 8,
wherein the connection mechanism has a filter.

10. A set for a cell encapsulation device with a suction tube, comprising:
the cell encapsulation device with a suction tube according to any one of claims 1 to 9, and
a rack accommodating the cell encapsulation device with a suction tube.

11. The set for a cell encapsulation device with a suction tube according to claim 10,
wherein the rack has a U-shaped locking portion.

12. A method for encapsulating cells, using the cell encapsulation device with a suction tube according to any one of claims 1 to 9.
